(19) Europäisches Patentamt
European Patent Office
Office européen des brevets

(11) Veröffentlichungsnummer : **0 003 515**
**B1**

(12) # EUROPÄISCHE PATENTSCHRIFT

(45) Veröffentlichungstag der Patentschrift : **11.02.81**

(21) Anmeldenummer : **79100167.0**

(22) Anmeldetag : **19.01.79**

(51) Int. Cl.³ : **C 07 D307/00**, C 07 D307/93, A 23 L 1/226, C 11 B 9/00// C07D303/14, C07D303/32

(54) **Neue Megastigman-4,7-oxyde (I) und neue 7-Oxabicyclo(3.3.0)-octane (II), Verfahren zur Herstellung von (I) und (II), Verwendung von (I) und (II) als Riech- und/oder Geschmackstoffe sowie Riech- und/oder Geschmackstoffkompositionen mit einem Gehalt an (I) oder (II).**

(30) Priorität : **09.02.78 CH 1449/78**
**09.02.78 CH 1449/78**

(43) Veröffentlichungstag der Anmeldung : **22.08.79 (Patentblatt 79/17)**

(45) Bekanntmachung des Hinweises auf die Patenterteilung : **11.02.81 Patentblatt 81/06**

(84) Benannte Vertragsstaaten : **CH DE FR GB NL**

(56) Entgegenhaltungen :
**CHEMICAL ABSTRACTS, Vol. 81, Nr. 15, Oktober 14. 1974, Columbus, Ohio, U.S.A., YU.V. TOMILOV et al. : « Acylation of norcarane by cationoid complexes », Seite 427, Zusammenfassung Nr. 91109d**

(73) Patentinhaber : **L. Givaudan & Cie Société Anonyme**
**Patentdienst Postfach**
**CH-4002 Basel (CH)**

(72) Erfinder : **Kaiser, Roman**
**Weidstrasse 6**
**CH-8610 Uster (CH)**

(74) Vertreter : **Urech, Peter, Dr., et al**
**Grenzacherstrasse 124, Postfach 601**
**CH-4002 Basel (CH)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

EP 0 003 515 B1

Neue Megastigman-4,7-oxyde (I) und neue 7-Oxabicyclo [3.3.0]-octane (II), Verfahren zur Herstellung von (I) und (II), Verwendung von (I) und (II) als Riech- und/oder Geschmackstoffe sowie Riech- und/oder Geschmackstoffkompositionen mit einem Gehalt an (I) oder (II)

Die Erfindung betrifft neue Riech- und/oder Geschmackstoffe, nämlich Verbindungen der allgemeinen Formeln

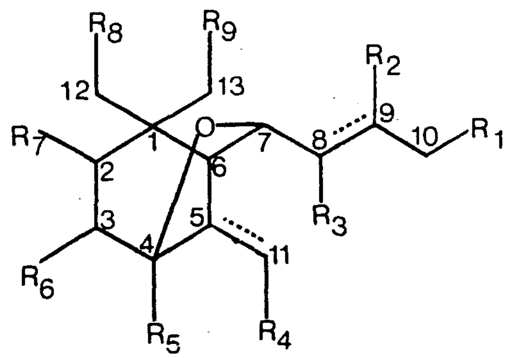

und

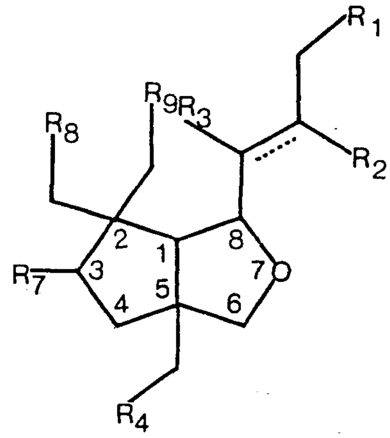

I II

worin $R_1$ und $R_2$ Wasserstoff, Methyl, Propyl, Vinyl, Propenyl oder Allyl und $R_3$, $R_4$, $R_5$, $R_6$, $R_7$, $R_8$, $R_9$ Wasserstoff, Methyl oder Aethyl darstellen, und die gestrichelten Linien eine fakultative Bindung, bzw. im Falle von $R_1$ und/oder $R_2$ = Propenyl, Vinyl oder Allyl eine obligatorische Bindung darstellen, und die 5,11-Bindung in I nur gesättigt ist, wenn der Rest des Moleküls gesättigt ist.

Die bevorzugten Verbindungen sind insbesondere die Jonon- und Ironderivate und die Verbindungen, in denen $R_2$ und/oder $R_3$ = Methyl oder $R_1$ und/oder $R_8$ und/oder $R_9$ = Methyl.

Die Erfindung betrifft auch ein Verfahren zur Herstellung dieser neuen Verbindungen der Formeln I und II. Es ist dadurch gekennzeichnet, dass man eine Verbindung der allgemeinen Formeln

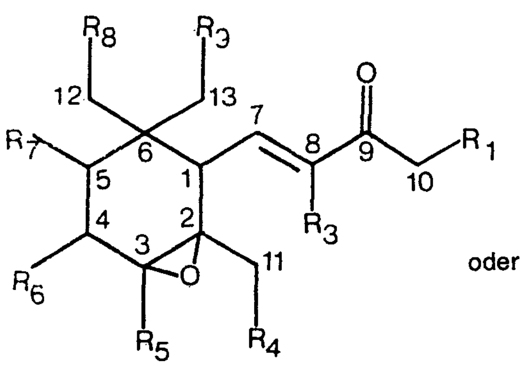

oder

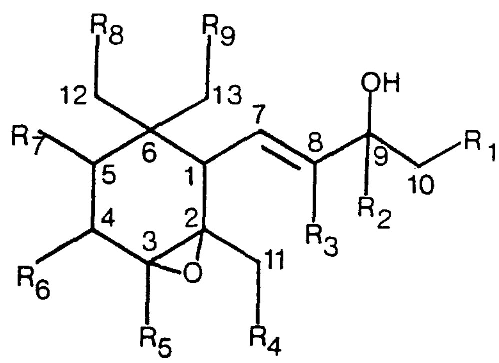

III IV

worin $R_1$ bis $R_9$ obige Bedeutung besitzen, mit einem Aluminium-sec.-alkoholat, im Falle von III unter reduzierenden Bedingungen, behandelt.

Die in Frage kommenden Aluminium-sec.-alkoholate sind insbesondere die mit 3-6 C-Atomen.

Das bevorzugte Reagens, mit dem die Ausgangsmaterialien umgesetzt werden, ist sowohl im Falle der Verbindung III wie IV Aluminiumisopropylat. Weiter sind insbesondere Aluminium-sec.-butylat und Aluminium-sec.-amylat geeignet.

Wenn III als Ausgangsmaterial verwendet wird, arbeitet man unter reduzierenden Bedingungen, was auf einfache Weise durch Verwendung eines alkoholischen Lösungsmittels, wie Isopropanol, sek. Butanol, sec. Amylalkohol etc. bewerkstelligt werden kann.

Zur Umsetzung der Ausgangsmaterialien der Formel IV können prinzipiell die gleichen Reagens-Lösungsmittel-Systeme verwendet werden. In diesem Fall muss das System aber nicht zwingend reduzierende Eigenschaften besitzen, sodass an Stelle von Isopropanol, usw. auch aprotische Lösungsmittel wie Toluol, Xylol, usw. treten können.

Die geeigneten Reaktionstemperaturen liegen zwischen 100 und 150 °C. Die Wahl des Lösungsmittels und der Reaktionstemperatur bestimmen die notwendige Reaktionsdauer. Wird die Umsetzung der Verbindungen der Formel III oder IV beispielsweise unter Verwendung von Aluminiumisopropylat/Isopropanol bei 110 °C vorgenommen, beträgt die Reaktionszeit z.B. 4 bis 6 Stunden. Bei einer Reaktionstemperatur von 130 °C verkürzt sich die Reaktionszeit in diesem Fall auf 1 bis 1,5 Stunden. Wird die Umsetzung der Verbindungen der Formel IV unter Verwendung von Aluminiumisopropylat in siedendem Toluol vorgenommen, beträgt die Reaktionsdauer 10 bis 15 Stunden, in siedendem Xylol dagegen verkürzt sie sich auf 5 bis 10 Stunden.

Die Verbindungen IV sind, in an sich bekannter Weise, aus den Verbindungen III, z.B. durch Reduktion mit $NaBH_4$ oder $LiAlH_4$, zugänglich.

Die Reaktionsprodukte werden zweckmässigerweise durch einfache Destillation gereinigt. Es resultiert auf diese Weise jeweils ein Gemisch von I + II, in dem der Anteil an II ca. 0,5 bis 20 % ausmacht.

Diese als Reaktionsprodukte anfallenden Gemische der Verbindungen I und II brauchen üblicherweise nicht in die Komponenten aufgetrennt werden, da die (als Nebenprodukte anfallenden) Verbindungen der Formel II ähnliche organoleptische Eigenschaften aufweisen wie die Verbindungen I. Es ist daher wirtschaftlicher, die Gemische einzusetzen.

Eine Auftrennung ist aber möglich, z.B. mit Hilfe der Säulenchromatographie oder präparativen Gaschromatographie.

Sowohl die Doppelbindungen der Seitenkette als auch die semicyclische Doppelbindung der primären Reaktionsprodukte können gegebenenfalls hydriert werden.

Durch selektive Hydrierung der — reaktiveren-Doppelbindung in der Seitenkette von I bzw. II — z.B. mittels $H_2$/Pt-IV-Oxyd in Aethanol als Lösungsmittel — resultieren die Megastigm-5(11)-en-4,7-oxyde (I) bzw. die 7-Oxa-bicyclo [3.3.0]-octanderivate II.

Durch erschöpfende Hydrierung — z.B. mittels $H_2$ in Anwesenheit eines Katalysators, wir Pt-IV-oxyd oder Palladium, in einem Lösungsmittel wie Aethanol, Hexan, Essigester usw. resultieren schliesslich die Megastigman-4,7-oxyde vom Typus I bzw. wiederum die 7-Oxa-bicyclo-[3.3.0]-octanderivate vom Typus II.

Die Verbindungen I und II stellen farblose bis leicht gelblich gefärbte Flüssigkeiten oder niedrigschmelzende kristalline Substanzen dar, sind unlöslich in Wasser, aber löslich in organischen Lösungsmitteln, wie z.B. Alkoholen, Aethern, Ketonen, Estern, Kohlenwasserstoffen und halogenierten Kohlenwasserstoffen.

Die Verbindungen I und II weisen besondere organoleptische Eigenschaften auf, auf Grund derer sie sich vorzüglich als Riech- und/oder Geschmackstoffe eignen.

Die Erfindung betrifft demgemäss auch die Verwendung von I und II als Riech- und/oder Geschmackstoffe.

Die Verbindungen I und II zeichnen sich, wie aus der Tabelle I leicht ersichtlich, durch ein sehr breites Spektrum von Geruchs-bzw. Geschmacksnoten aus.

TABELLE 1

Beispiele von Verbindungen der Formeln I und II

| | | |
|---|---|---|
| Megastigma-5(11),8-dien-4,7-oxyd | 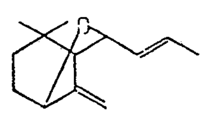 1 | erfrischend, fruchtig, würzig, blumig, camphrig, Aspekte der Kopfnote von Geraniumöl |
| 2,2,5-Trimethyl-8-[prop-1′-enyl]-7-oxa-bicyclo [3.3.0]-octan | 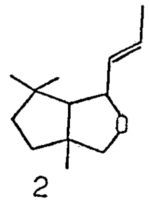 2 | angenehm blumig (Richtung Rose), würzig, fruchtig |
| Megastigm-5(11)-en-4,7-oxyd | 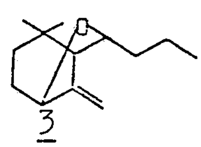 3 | camphrig, fruchtig, rosig mit würzigem Aspekt |
| Megastigman-4,7-oxyd | 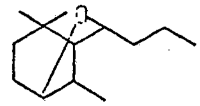 4 | fruchtig, blumig mit leicht erdigem Aspekt |

| | | |
|---|---|---|
| 2-Methyl-megastigma-5(11),8-dien-4,7-oxyd | 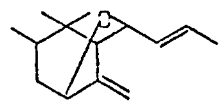 5 | Iriswurzeln, gewisse Aspekte von Jasmin und Schwarztee, weich und balsamisch |
| 2-Methyl-megastigm-5(11)-en-4,7-oxyd | 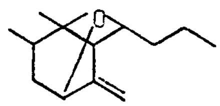 6 | Iriswurzeln, süss, camphrig |
| 8-Methyl-megastigma-5(11),8-dien-4,7-oxyd | 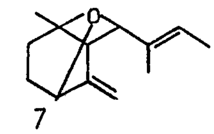 7 | holzig, camphrig, fruchtig |
| 2,2,5-Trimethyl-8-[1'-methyl-prop-1'-enyl]-7-oxa-bicyclo [3.3.0]-octan | 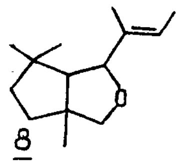 8 | edelholzartig, mit fruchtig-blumigem Aspekt |
| 8-Methyl-megastigm-5(11)-en-4,7-oxyd | 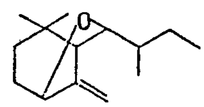 9 | fruchtig, holzig, camphrig mit leicht erdigem Aspekt |
| 9-Methyl-megastigma-5(11),8-dien-4,7-oxyd | 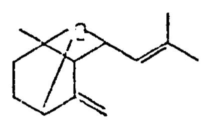 10 | trockenes Holz, cedrig, leicht camphrig |
| 2,2,5-Trimethyl-8-[2'-methyl-prop-1'-enyl]-7-oxa-bicyclo [3.3.0]-octan | 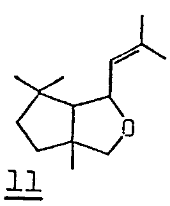 11 | trockenes Holz, camphrig, Aspekte von Zeder und Costus |
| 9-Methyl-megastigm-5(11)-en-4,7-oxyd | 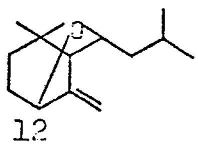 12 | würzig, trocken, camphrig |
| 9-Aethyl-megastigma-5(11),8-dien-4,7-oxyd | 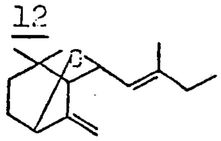 13 | balsamisch, holzig, trocken |
| 10-Methyl-megastigma-5(11),8-dien-4,7-oxyd | 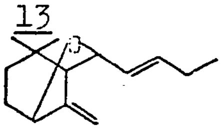 14 | balsamisch, grün, fruchtig, camphrig |
| 10-Methyl-megastigm-5(11)-en-4,7-oxyd | 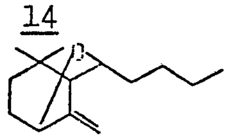 15 | fruchtig, angenehm grün |

| | | |
|---|---|---|
| 10-Allyl-megastigma-5(11),8-dien-4,7-oxyd | 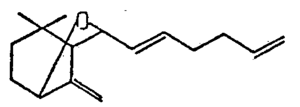 16 | metallisch, fruchtig Richtung Ananas, leicht blumig |
| 11-Methyl-megastigma-5(11),8-dien-4,7-oxyd | 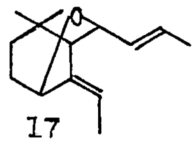 17 | süss, honigartig, teeartig, rosig |
| 4,11-Dimethyl-megastigma-5(11),8-dien-4,7-oxyd | 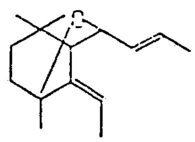 18 | sehr natürlich, aromatisch, teeartig, Aspekte der Kopfnoten von Geranium- und Rosenöl |
| 12/13-Methyl-megastigma-5(11),8-dien-4,7-oxyd | 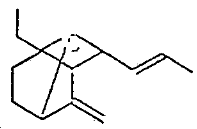 19 | grün, grapefruitartig, rosig, aldehydisch, camphrig |
| 9-Vinyl-megastigma-5(11),8-dien-4,7-oxyd | 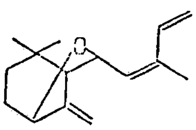 20 | grün, balsamisch, terpenisch |

Die erfindungsgemäss als Riech- und/oder Geschmackstoffe verwendeten Verbindungen können demgemäss beispielsweise zur Parfümierung bzw. Aromatisierung von Produkten, wie Kosmetika (Seifen, Salben, Pudern, Zahnpasten, Mundwässern, Desodorantien, Shampoos, Lotionen, Eau de Toilette, Eau de Cologne, Extraits, etc.), Waschmitteln, Detergentien, Raucherartikeln, bzw. Nahrungsmitteln, Genussmitteln und Getränken dienen, wobei die Verbindungen vorzugsweise nicht allein, sondern in Form von Kompositionen mit andern Riech-bzw. Geschmackstoffen eingesetzt werden. Solche Riech-bzw. Geschmackstoffkompositionen mit einem Gehalt an Verbindungen I und/oder II und deren auf an sich bekannte Art erfolgende Herstellung (Zugabe von I und/oder II zu bekannten Riech-bzw. Geschmackstoffkompositionen oder Vermischung von I mit als Bestandteil von Riech-bzw. Geschmackstoffkompositionen geeigneten natürlichen oder synthetischen Verbindungen oder Gemischen) bilden ebenfalls Gegenstand der Erfindung.

Als Riech- und/oder Aromastoffe eignen sich die Verbindungen der Formeln I und II bzw. ihre Gemischeinsbesondere die Gemische mit hohem Gehalt an I-aufgrund ihrer wertvollen olfaktischen Eigenschaften, insbesondere in Kombination mit einer umfangreichen Reihe von natürlichen und synthetischen Riechstoffen bzw. Aromastoffen wie z.B.

— Galbanumöl, Mastixöl, Vetiveröl, Patchouliöl, Patcouliblätteröl, Sandelholzöl, Cedernöl, Fichtenöl, Lorbeeröl, Costuswurzelöl, Calmusöl, Baummoos absolue, Basilikumöl, Beifussöl, Kamillenöl, Wermutöl, Wurmsamenöl, Selleriesamenöl, Angelikasamenöl, Sternanisöl, Thymianöl, Rosmarinöl, Lavendelöl, Lavandinöl, Aspiköl, Salbeiöl, Petitgrainöl, Neroliöl, Bergamotteöl, Citronenöl, Mandarinenöl, Orangenöl, Grapefruitöl, Geraniumöl, Benzoe-resinoid, Melilotus absolue, Jasmin absolue, Rosenöl, Ylang-Ylang-Oel, Canangaöl, Corianderöl, Cassie absolue, Narzissen absolue, Verveine absolue oder -öl, Veilchenblätter absolue, Tuberosen absolue usw.,

— mit Aldehyden wie Hydroxycitronellal, Zyklamenaldehyd, p-tert. Butyl-α-methylhydrozimtaldehyd, alpha-Hexylzimtaldehyd, 3,5-Dimethyl-cyclohex-3-en-1-yl-carboxaldehyd, Citral, Citronellal, 2,6-Dimethyl-6-hepten-1-al, Isovaleraldehyd, trans-2-Hexenal, Sorbinaldehyd, trans-2-Octenal, n-Octanal, n-Nonanal, trans-2-cis-6-Nonadienal, 2,4-Decadienal, Methylnonyl-acetaldehyd usw.,

— mit Ketonen wie alpha-Ionon, beta-Ionon, Acetanisol, 4-(para-Hydroxyphenyl)-2-butanon, Campher, Menthon, Carvon, Pulegon usw.,
— mit Acetalen und Ketalen wie Phenylacetaldehyddimethylacetal, Phenylacetaldehyd-glycerinacetal, 2-Methyl-1,3-dioxolan-2-äthylacetat, Capronaldehyddimethylacetal usw.,
— mit Aethern wie Eugenolmethyläther, Methyl-1-methyl-cyclododecyläther, Anethol, Estragol, usw.,
— mit phenolischen Körpern wie Eugenol, Isoeugenol, Creosol usw.,
— mit Alkoholen wie Butanol, cis-3-Hexanol, trans-2-cis-6-Nonadienol, cis-6-Nonenol, Linalool, Geraniol, Nerol, Citronellol, Nerolidol, Farnesol, Benzylalkohol, Phenyläthylalkohol, Zimtalkohol usw.,
— mit Estern wie Methyldihydrojasmonat, Linalylacetat, Geranylacetat, Cedrylacetat, Vetiverylacetat, Aethylisovalerat, Aethylcaproat, para-tert.-Butylcyclohexyl-acetat, ortho-tert.-Butylcyclohexylacetat, [4-(4-Methyl-3-pentenyl)-3-cyclohexen-1-yl] methylacetat, Benzylacetat, Benzylsalicylat, Styrallylacetat, Aethyl-α-methylphenylglycidat, Aethyl-trans-2-hexenoat, Aethyl-trans-2-octenoat usw.,
— mit Lactonen wie γ-Undecalacton, γ-Decalacton, γ-Nonalacton, γ-Decalacton, γ-Octalacton, Cumarin usw.,
— mit Säuren wie Milchsäure, Buttersäure, α-Methylbuttersäure, trans-2-Hexensäure, trans-2-Octensäure usw.,
— mit moschus- und ambraartig riechenden Körpern wie Aethylenbrassylat, 4-Acetyl-6-tert.-butyl-1,1-dimethyl-indan, 12-Oxahexadecanolid, 8α,12-Oxido-13,14,15,16-tetranorlabdan usw.,
— mit schwefelhaltigen Verbindungen wie p-Menthan-8-thiol-3-on, Dimethylsulfid und anderen Sulfiden und Disulfiden usw.,
— mit stickstoffhaltigen Verbindungen wie Methylanthranilat, Indol, Isobutylchinolin, verschiedenen Pyrazinen usw.

Wie aus den weiter unten folgenden Anwendungsbeispielen ersichtlich ist, lassen sich mit den Megastigman-Derivaten der Formel I, die jeweils zu ca. 0.5 bis 20 % der entsprechenden 7-Oxa-bicyclo [3.3.0]-octan-Derivate der Formel II begleitet sind, bzw. mit II, äusserst interessante Effekte erzielen.

Neben diesen orginellen Effekten in Riechstoffkompositionen, beispielsweise vom Typ Chypre, Cologne, Tabak, Holz, Rose bzw. allgemein blumiger Richtung, ist es aber auch möglich, mit den erfindungsgemässen Verbindungen neuartige Parfümerie-Komplexe herzustellen. So besitzt z.B. eine 1 :1-Mischung von Geraniol und dem Megastigma-5(11), 8-dien-4,7-oxyd *1* einen intensiven und sehr diffusiven Geruch, der an Rhodinol und typische Kopfnoten von Rosenöl und Geraniumöl erinnert.

Daher eignet sich dieser Komplex vorzüglich zur Herstellung von synthetischen Rosen- und Geraniumölen.

Weiter vermag die gleiche Verbindung Komplexe aus Linalool/Geraniol und Citral enorm aufzuwerten, indem der Eindruck erweckt wird, als ob bei der Formulierung so wertvolle Naturprodukte wie Rosenöl und Citronenöl mitverwendet worden seien.

Auch vermag, z.B. das 2-Methyl-megastigma-5(11), 8-dien-4,7-oxyd *5* die einzelnen Elemente eines Gemisches von Bergamotteöl, Sandelholzöl und Methyldihydrojasmonat in geradzu idealer Weise untereinander zu verbinden und den gesamten Komplex sehr vornehm erscheinen zu lassen, so dass dieser für den Einsatz in der Luxus-Parfümerie prädestiniert ist.

Das 8-Methyl-megastigma-5(11), 8-dien-4,7-oxyd *7* wird dagegen mit Vorteil zur Abrundung und Verstärkung von Holzkomplexen - die z.B. aus Sandelholzöl, Patchouliöl, Vetiveröl und Cedrylacetat zusammengesetzt sein können - verwendet.

Die Konzentration der Verbindungen I und II kann je nach dem Verwendungszweck innerhalb weiter Grenzen variieren, beispielsweise zwischen etwa 0,01 (Detergentien) und etwa 15 Gew. % (alkoholische Lösungen). In Parfumbasen bzw. Konzentraten können die Konzentrationen selbstverständlich auch höher liegen. Die Parfumbasen können in üblicher Weise zur Parfümierung von Eaux de Cologne, Eaux de toilette, Lotionen, Crèmes, Shampoos, Seifen und Detergentien, etc. verwendet werden.

Als Geschmackstoffe können die Verbindungen I und II beispielsweise zur Erzeugung bzw. Verbesserung, Verstärkung, Steigerung oder Modifizierung von Frucht- oder Beerenaromen in Nahrungsmitteln (Joghurt, Süsswaren, etc.) ; in Genussmitteln (Tee, etc.), Getränken (Limonaden etc.) und Tabak verwendet werden.

Die ausgeprägten geschmacklichen Qualitäten der Verbindungen I und II ermöglichen die Verwendung in geringen Konzentrationen. Eine geeignete Dosierung umfasst beispielsweise den Bereich von 0,1 ppm-100 ppm, vorzugsweise von 1 ppm-20 ppm im Fertigprodukt, d.h. dem aromatisierten Nahrungsmittel, Genussmittel oder Getränk.

Bei der Aromatisierung von beispielsweise Tabak kann die Dosierung jedoch auch höher liegen und einen grösseren Bereich bestreichen, beispielsweise den Bereich von 1 bis 1000 ppm, vorzugsweise 50 bis 700 ppm.

In der folgenden Tabelle sind einige Effekte zusammengestellt, wie sie sich beispielsweise mit den Aethern I erzielen lassen.

TABELLE

| Verbindung | Aroma | Dosierung | Effekt |
|---|---|---|---|
| *1, 2, 17, 18* | Himbeer Tee | ppm im Fertigprodukt 0,1-30 ppm insb. 0,5- 4 ppm | grössere Natürlichkeit des Fruchtcharakters, volles Aroma der reifen Früchte bzw. typisches Teearoma, indem holzige, herb-frische, z.T. auch blumige Noten verstärkt auftreten |
| *19* | Citrus, insb. Grapefruit | ppm im Fertigprodukt 0,1-100 ppm insb. 1 - 20 ppm | sehr natürlicher Fruchtcharakter, verbesserte Kopfnote |
| *16* | Ananas | ppm im Fertigprodukt 0,1-100 ppm insb. 1 - 20 ppm | Charakter der frischen Frucht wird unterstrichen, Abrundungseffekt |
| *1, 2, 5, 17, 18* | Tabak | ppm im Tabak 1 -1 000 ppm insb. 50- 700 ppm | beim Verrauchen angenehmer Kühleffekt, bedeutend angenehmeres und milderes Aroma |

Die Verbindungen können auf übliche Weise mit den für Geschmackstoffkompositionen verwendeten Bestandteilen vermischt bzw. solchen Aromen zugesetzt werden. Unter den erfindungsgemäss verwendeten Aromen werden Geschmackstoffkom positionen verstanden, die sich auf an sich bekannte Art verdünnen bzw. in essbaren Materialien verteilen lassen. Sie können nach an sich bekannten Methoden in die üblichen Gebrauchsformen, wie Lösungen, Pasten oder Pulver übergeführt werden. Die Produkte können sprühgetrocknet, vakuumgetrocknet oder lyophilisiert werden.

Für die Herstellung solcher üblicher Gebrausformen kommen beispielsweise folgende Trägermaterialien, Verdickungsmittel, Geschmackstoffverbesserer, Gewürze und Hilfsingredientien, etc. in Frage : Gummi arabicum, Tragant, Salze oder Brauereihefe, Alginate, Carrageen oder ähnliche Absorbentien ; Maltol, Gewürzoleoresine, Raucharomen ; Gewürznelken, Natriumcitrat ; Mononatriumglutamat, Dinatrium-inosin-5'-monophosphat (IMP), Dinatriumguanosin-5-phosphat (GMP) ; oder spezielle Aromastoffe, Wasser, Aethanol, Propylenglykol, Glycerin.

## Beispiel 1

Megastigma-5(11), 8-dien-4,7-oxyd *1* und 2,2,5-Trimethyl-8-[prop-1'-enyl]-7-oxabicyclo [3.3.0]-octan *2*

a) Zu einer Lösung von 63,0 g (0,30 Mol) α-Jononepoxyd (P. Karrer, H. Sturzinger, Helv. chim. Acta *29*, 1829 (1946)) in 250 ml Aethanol liess man im Verlaufe von 20 Minuten unter leichter Kühlung eine Lösung von 3,80 g (0,1 Mol) Natriumborhydrid in 135 ml Aethanol so zutropfen, dass eine Reaktionstemperatur von 15° nicht überschritten wurde. Anschliessend rührte man noch während 20 Minuten bei Raumtemperatur, verdünnte das Reaktionsgemisch mit 1 l Aether, wusch die Aetherphase bis zum Neutralpunkt, trocknete mit Natriumsulfat und engte ein. Es resultierten 58 g rohes α-Jonolepoxyd, von denen 56 g für die nächste Stufe eingesetzt wurden.

56,0 g (~0,26 Mol) α-Jonolepoxyd löste man, zusammen mit 26,5 g (0,13 Mol) Aluminiumisopropylat in 170 ml Isopropanol, brachte das Gefäss in ein Oelbad von 160° und destillierte 9/10 des eingesetztes Isopropanols so über eine 20 cm-Vigreux-Kolonne ab, dass die Temperatur des Reaktionsgemisches auf 130° stieg. Die zurückbleibende viskose Masse wurde anschliessend während 1 1/2 Studen bei einer Oelbadtemperatur von 145° (≙ einer Temp. des Reaktionsgemisches von 130-135°) gerührt, dann vorsichtig mit den abdestillierten 150 ml Isopropanol versetzt, das erhaltene Reaktionsgemisch abgekühlt und mit Aether extrahiert. Die Aetherphase wurde dreimal mit verdünnter Salzsäurelösung und anschliessend mit Wasser bis zum Neutralpunkt gewaschen, getrocknet und eingeengt. Die Destillation des Rohproduktes (48 g) über eine 20 cm-Widmer-Kolonne ergab 14,3 g (≙ 28 %) olfaktisch gutes Produkt vom Sdp. 51-56°/0,1 mmHg, das 90 % *1* und *2* im Verhältnis von 10 : 1 enthielt.

b) 31,2 g (0,15 Mol) α-Jononepoxyd löste man zusammen mit 15,3 g (0,075 Mol) Aluminiumisopropylat in 90 ml Isopropanol und verfuhr im weiteren wie unter a) beschrieben. Die Destillation des erhaltenen Rohproduktes (27 g) ergab 8,7 g (Δ 30,2 %) olfaktisch gutes Produkt, welches >90 % *1* und *2* im Verhältnis von 10 : 1 enthielt. 3,5 g des 10 : 1-Gemisches wurden mittels präparativer Gaschromatographie (GC) aufgetrennt.

Spektrale Daten

*1.* IR : 1680, 1222, 1154, 1078, 1054, 1020, 982, 962, 940, 894 $cm^{-1}$

NMR : 0,94+1,02 (je s, je 3 H) ; 1,67 (d,J~6Hz, $CH_3$-C(9)) ; 1,92 (s, 1H-C(6)) ; 4,36 (d,J~4Hz, 1H-C(4)) ; 4,50 (d,J~7Hz, 1H-C(7)) ; 4,76+4,97 (je s, je 1H-C(11)) ; 5,37 (dxd,J(8,9)~15Hz, J(7,8)~7Hz abgelesen nach w2→1,67 ppm, 1H-C(8)) ; 5,67(dxq,J(8,9)~15Hz, J(9,10)~6Hz, 1H-C(9)) ; δ ppm

MS : 192 ($M^+$,9), 177 (15), 163 (8), 159 (10), 122 (74), 107 (100), 93 (42), 91 (25), 79 (30), 41 (19)

*2.* IR : 1680, 1082, 1055, 978, 966 $cm^{-1}$

NMR : 1,00 (2s, zusammenfallend, 6H) ; 1,28 (s, $CH_3$-C(5)) ; 1,72 (d,J~5,5Hz 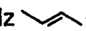 $CH_3$) ; 3,38+3,68 (je d, J gem~8,5Hz, 2H-C(6)) ; 4,04 (dxd, J je~7Hz, 1H-C(8)) ; 5,56 (dxd, $J_1$~15Hz, $J_2$~7Hz, 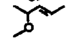 ) ; 5,80 (dxq, $J_1$~15Hz, $J_2$ 5,5Hz, 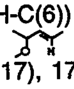 ) δ ppm

MS : 194 ($M^+$, 17), 179 (21), 125 (100), 109 (66), 95 (22), 81 (33), 69 (43), 67 (42), 55 (34), 41 (50), und weitere typische Fragmente bei m/e 82 (28), 68 (33)

## Beispiel 2

Megastigm-5(11)-en-4,7-oxyd *3* und 2,2,5-Trimethyl-8-propyl-7-oxa-bicyclo [3.3.0]-octan *3a*

1,0 g (5,2 mMol) *1* (+ *2*) wurden in 10 ml Aethanol gelöst und in Anwesenheit von 10 mg Pt-IV-Oxyd bis zur Aufnahme von 140 ml Wasserstoff hydriert. Die Kugelrohrdestillation des nach der Aufarbeitung erhaltenen Rohproduktes ergab 0,87 g rund 90 %iges Produkt *3* und 2,2,5-Trimethyl-8-propyl-7-oxa-bicyclo [3.3.0]-octan *3a* im Verhältnis 10 : 1. Zur Characterisierung wurde eine Probe von *3* mittels präparativer GC nachgereinigt.

IR : 1685, 1155, 1065, 1058, 972, 968, 890 $cm^{-1}$

NMR : 0,96 (2s, zusammenfallend, 6H) ; 0,94 (t, schlecht aufgelöst, $CH_3$-C(9)) ; 4,10 (m, 1H-C(4)) ; 4,25 (m, 1H-C(7)) ; 4,72+4,91 (je s, je 1H-C(11)) δ ppm

MS : 194 ($M^+$, 4) 179 (23), 151 (46), 135 (17), 122 (37), 107 (77), 95 (100), 81 (81), 69 (39), 55 (35), 41 (56) und weitere typische Fragmente bei m/e 109 (39), 93 (42), 91 (36), 79 (41), 67 (35)

## Beispiel 3

Megastigman-4,7-oxyd *4* 2,2,5-Trimethyl-8-propyl-7-oxa-bicyclo [3.3.0]-octan *4a*

3,7 g (19 mMol) *1* (+ *2*) wurden in 25 ml Aethanol gelöst und in Anwesenheit von 60 mg Pt-IV-oxyd bis zur Aufnahme von 940 ml Wasserstoff hydriert. Die Kugelrohrdestillation des nach der Aufarbeitung erhaltenen rohproduktes ergab 3,35 g über 90 % *4 und* 2,2,5-Trimethyl-8-propyl-7-oxa-bicyclo [3.3.0]-octan *4a* im Verhältnis 10 : 1. Eine mittels präparativer GC gereinigte Probe von *4* zeigte folgende spektrale Daten.

IR : 1238, 1163, 1142, 1072, 1042, 982, 969, 954 $cm^{-1}$

NMR : 0,91+1,10 (je s, je 3H) ; 0,94 (t,J~6,5Hz, $CH_3$-C(9)) ; 2,28 (2m, 1H-C(5) und 1H-C(6)) ; 4,00 (2m, überlagert, 1H-C(4) und 1H-C(7)) δ ppm

MS : 196 ($M^+$,1) 153 (100), 135 (24), 109 (50), 97 (19), 95 (51), 81 (15), 69 (27), 55 (22), 43 (12), 41 (25)

## Beispiel 4

2-Methylmegastigma-5(11),8-dien-4,7-oxyd *5* und 2,2,3,5-Tetramethyl-8-[prop-1'-enyl]-7-oxa-bicyclo [3.3.0]-octan *5a*

Zu einem auf 0° gekühlten Gemisch von 206,0 g (1 Mol) α-Iron und 198 g wasserfreiem Natriumacetat in 1 1 Methylenchlorid wurden unter Rühren 198 g (1,1 Mol) 40 %ige Peressigsäure im Verlaufe von 40 Minuten so zugetropft, dass die Reaktionstemperatur zwischen 10 und 15° lag. Anschliessend entfernte man die externe Kühlung und rührte das Reaktionsgemisch während 4 Stunden. Im Verlaufe der ersten Stunde stieg die Temperatur des Reaktionsgemisches auf 30° und fiel hierauf wieder langsam auf Raumtemperatur. Man entfernte das Natriumacetat durch Filtration, wusch die klare Lösung je dreimal mit Wasser, Natriumsulfitlösung, Natriumbicarbonatlösung und nochmals mit Wasser, trocknete mit Natriumsulfat und engte ein. Es resultierten 215 g Rohprodukt, die gemäss GC>92 % α-Ironepoxyd enthielten. Durch Destillation über eine 30 cm-Widmer-Kolonne wurden 190 g α-Ironepoxyd (≙ 86 %) vom Sdp. 100-101°/0,06 mmHg erhalten.

55,5 g (0,25 Mol) α-Ironepoxyd löste man zusammen mit 38,4 g (0,19 Mol) Aluminiumisopropylat in 180 ml Isopropanol und bediente sich im weiteren der im Beispiel 1 beschriebenen Methodik. Die Destillation des Rohproduktes (54 g) über eine 10 cm-Widmer-Kolonne ergab 13,2 g (≙25,6 %) olfaktisch gutes Produkt vom Sdp. 60-63°/0,2 mmHg, welches zu >90 % aus *5* und 2,2,3,5-Tetramethyl-8-[prop-1'-enyl]-7-oxa-bicyclo [3.3.0]-octan *5a* im Verhältnis ~20 : 1 bestand.

Spektrale Daten von 5

IR : 1680, 1040, 1030, 980, 968, 910, 890 $cm^{-1}$

NMR : 0,78+1,02 (je s, je 3H) ; 1,00 (d,J~6,5Hz, $CH_3$-C(2)) ; 1,67 (d,J~6Hz, $CH_3$-C(9)) ; 1,92 (s, 1H-C(6)) ; 4,34 (d,J~4Hz, 1H-C(4) ; 4,50 (d,J~7Hz, 1H-C(7)) ; 4,76+4,96 (je s, je 1H-C(11)) ; 5,26-5,86 (m, 1H-C(8) und 1H-C(9), Signalanalyse vergl. NMR von *1*)

MS : 206 ($M^+$,1), 136 (50), 121 (100), 109 (36), 107 (69), 93 (45), 79 (39), 77 (27), 67 (28), 55 (26), 41 (45)

## Beispiel 5

2-Methylmegastigm-5(11)-en-4,7-oxyd *6* und 2,2,3,5-Tetramethyl-8-propyl-7-oxa-bicyclo [3.3.0]-octan *6a*

2,06 g (10 mMol) *5* wurden in 20 ml Aethanol gelöst und in Anwesenheit von 20 mg Pt-IV-oxyd bis zur Aufnahme von 270 ml Wasserstoff hydriert. Die Kugelrohrdestillation des nach der Aufarbeitung erhaltenen Rohproduktes ergab 1,75 g rund 88 %iges *6* und 2,2,3,5-Tetramethyl-8-propyl-7-oxa-bicyclo [3.3.0]-octan *6a* im Verhältnis ~20 : 1. Zur Charakterisierung wurde eine Probe von *6* mittels präparativer GC nachgereinigt.

IR : 1690, 1078, 1062, 1043, 1032, 890 $cm^{-1}$

NMR : 0,78+0,97 (je s, je 3H) ; 0,77 (d,J~6,5Hz, $CH_3$-C(2)) ; 0,9 (t, schlecht aufgelöst, $CH_3$-C(9)) ; 1,85 (s, 1 H-C(6)) ; 4,05 (m, 1H-C(4)) ; 4,26 (m, 1H-C(7)) ; 4,72+4,90 (je s, je 1H-C(11)) δ ppm

MS : 208 ($M^+$,16), 193 (60), 165 (100), 149 (51), 136 (46), 121 (87), 109 (76), 95 (89), 81 (53), 69 (34), 55 (39), 41 (67) und weitere typische Fragmente bei m/e 167 (76), 137 (40), 123 (58), 107 (49)

## Beispiel 6

8-Methylmegastigma-5(11),8-dien-4,7 oxyd *7* und 2,2,5-Trimethyl-8-[1'-methylprop-1'-enyl]-7-oxabicyclo-[3.3.0]-octan *8*

Analog der im Beispiel 4 beschriebenen Epoxydation von α-Iron wurden 206,0 g (1 Mol) Isomethyl-α-jonon epoxydiert. Durch Destillation des Rohproduktes über eine 20 cm-Widmer-Kolonne wurden 194 g (> 88 %)> 94 % reines Isomethyl-α-jononepoxyd vom Sdp. 94-95°/0,06 mmHg erhalten.

80,0 g (0,36 Mol) Isomethyl-α-jononepoxyd löste man mit 36,8 g (0,18 Mol) Aluminiumisopropylat in 240 ml Isopropanol und bediente sich der im Beispiel 1 beschriebenen Methodik. Die Destillation des Rohproduktes (77 g) über eine 15 cm-Widmer-Kolonne ergab 28,1 g (≙38 %) olfaktisch gutes Produkt vom Sdp. 68-72°/0,09 mmHg, welches > 80 % *7* und *8* im Verhältnis von ungefähr 9 : 1 enthielt. 2,5 g des 9 : 1-Gemisches wurden mittels präparativer GC aufgetrennt.

Spektrale Daten von *7* und *8*

*7.* IR : 1680, 1060, 1055, 990, 968, 890 $cm^{-1}$

NMR : 0,98 + 1,06 (je s, je 3H) ; 1,56 (s, $CH_3$-C(8)) ; 1,60 (d, J ~ 6,5Hz, $CH_3$-C(9)) ; 1,94 (s, 1H-C(6)) ; 4,4-4,5 (1H-C (4) und 1H-C (7), überlagert) ; 4,68 und 4,94 (je s, je 1H-C(11)) ; 5,44 (m, 1H-C(9)).

MS : 206 ($M^+$, 2), 122 (57), 107 (100), 93 (41), 91 (24), 79 (38), 77 (17), 67 (13), 55 (21), 41 (29), 39 (14)

*8.* IR : 1680, 1082, 1060, 965, 827, 807 $cm^{-1}$

NMR : 0,97 (2s zusammenfallend, 6H) ; 1,28 (s, $CH_3C(5)$) ; 1,60 (d mit Feinaufspaltung, 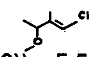 ) ; 1,66 (s, 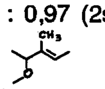 ) ; 3,36 + 3,68 (je d, J gem. ~ 8,5Hz, 2H-C(6)) ; 4,02 (d, J ~ 7Hz, 1H-C(8)) ; 5,50 (m, 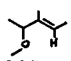 )

MS : 208 ($M^+$, 96), 193 (100), 139 (93), 109 (24), 95 (24), 82 (36), 69 (55), 68 (40), 55 (38), 41 (44)

## Beispiel 7

8-Methylmegastigm-5(11)-en-4,7-oxyd *9* und 2,2,5-Trimethyl-8- [1'-methylpropyl]-7-oxa-bicyclo [3.3.0]-octan *9a*

7,0 g (0,034 Mol) *7* (+ *8*) wurden in 70 ml Aethanol gelöst und in Anwesenheit von 70 mg Pt-IV-oxyd bis zur Aufnahme von 870 ml Wasserstoff hydriert. Die Kugelrohrdestillation des nach der Aufarbeitung erhaltenen Rohproduktes ergab 8,6 g rund 87 %iges *9* und 2,2,5-Trimethyl-8-[1'-methylpropyl]-7-oxa-bicyclo [3.3.0]-octan *9a* im Verhältnis ~ 9 : 1. Eine Probe von *9* wurde mittels präparativer GC nachgereinigt.

IR : 1685, 1059, 968, 890 $cm^{-1}$

NMR : 0,97 (2 s zusammenfallend, 6H) ; 0,75-0,95 (t, $CH_3$-C(9) und d $CH_3$-C(8), überlagert) ; 2,00 (s, 1H-C(6)) ; 3,80 und 4,29 (je m, 1H-C(4), 1H-C(7)) ; 4,71 + 4,91 (je s, je 1H-C(11)) δ ppm

MS : 208 ($M^+$, 1), 151 (10), 138 (14), 123 (54), 109 (19), 107 (35), 95 (100), 81 (36), 69 (18), 55 (20), 41 (45)

## Beispiel 8

9-Methylmegastigma-5(11), 8-dien-4,7-oxyd *10* und 2,2,5-Trimethyl-8-[2'-methylprop-1'-enyl]-7-oxabicyclo-[3.3.0]-octan *11*

Zu einer auf 0° gekühlten Grignard-Lösung, welche aus 7,5 g (0,31 Mol) Magnesiumspänen in 75 ml Aether und 46,9 g (0,33 Mol) Methyljodid in 180 ml Aether hergestellt wurde, liess man im Verlaufe von 20 Minuten eine Lösung von 50,0 g (0,26 Mol) α-Jonon in 150 ml Aether so zutropfen, dass die Reaktionstemperatur zwischen 10° und 20° lag. Anschliessend rührte man noch während 1 Stunde bei der Rückflusstemperatur des Aethers, kühlte auf 0° ab und versetzte das Reaktionsgemisch vorsichtig mit konzentrierter Ammoniumchloridlösung und zusätzlichen 500 ml Aether. Die Aetherphase wurde mit Wasser bis zum Neutralpunkt gewaschen, getrocknet und eingeengt. Es wurden 60,0 g Rohprodukt erhalten, welches > 95 % 9-Methyl-α-jonol (GC) enthielt und demzufolge direkt in dieser Form weiterverarbeitet wurde. Analog der Epoxydation von α-Iron (Beispiel 4) wurden aus 58,1 g (~ 0,26 Mol) 9-Methyl-α-jonol 68,0 g rohes 9-Methyl-α-jonol-epoxyd erhalten.

66,9 g (0,25 Mol) rohes 9-Methyl-α-jonolepoxyd löste man zusammen mit 26,5 g (0,13 Mol) Aluminiumisopropylat in 200 ml Isopropanol und wendete hierauf die unter Beispiel 1 beschriebene Methodik an. Die Destillation des Rohproduktes (64,0 g) über eine 20 cm-Widmer-Kolonne ergab 20,7 g (≙ 39 %) olfaktisch gutes Produkt vom Sdp. 67-71°/0,08 mmHg, das > 90 % *10* und *11* im Verhältnis von 8 : 1 enthielt. 2,2 g des 8 : 1-Gemisches wurden mittels präparativer GC aufgetrennt.

Spektrale Daten von *10* und *11*

*10.* IR : 1680, 1053, 1019, 982, 961, 889 $cm^{-1}$

NMR : 0,97 + 1,03 (je s, je 3H) ; 1,72 (2 zusammenfallende s, $2CH_3$-C(9)) ; 1,88 (s, 1H-C(6)) ; 4,36 (d, J ~ 4Hz, 1H-C(4)) ; 4,82 (d, J ~ 8Hz, 1H-C(7)) ; 4,76 + 4,94 (je s, je 1H-C(11) ; 5,12 (d mit Feinaufspaltung J ~ 8Hz, 1H-C(8)) δ ppm

MS : 206 ($M^+$, 9), 191 (11), 150 (11), 122 (65), 107 (100), 93 (40), 91 (27), 85 (21), 79 (24), 55 (13), 41 (25)

*11.* IR : 1680, 1053, 1009, 978, 961 $cm^{-1}$

NMR : 0,90 + 0,98 (je s, je 3H) ; 1,23 (s, $CH_3$-C(5)) ; 1,73 + 1,74 (je s, 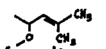 ) ; 3,31 + 3,65 (je d, J gem ~ 8Hz, 2H-C(6)) ; 4,20 (dxd, J je 8Hz, 1H-C(8)) ; 5,20 (d mit Feinaufspaltung, J ~ 8Hz, 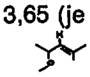 ) δ ppm

MS : 208 ($M^+$, 114), 193 (100), 139 (22), 109 (47), 95 (14), 85 (19), 82 (23), 69 (49), 68 (27), 55 (21), 41 (28)

## Beispiel 9

9-Methylmegastigm-5(11)-en-4,7-oxyd *12* und 2,2,5-Trimethyl-8-isobutyl-7-oxa-bicyclo [3.3.0]-octan *12a*

2,06 g (10 mMol) *10* (+ *11*) wurden in 20 ml Aethanol gelöst und in Anwesenheit von 20 mg Pt-IV-oxyd bis zur Aufnahme von 260 ml Wasserstoff hydriert. Die Kugelrohrdestillation des nach Aufarbeitung erhaltenen Rohproduktes ergab 1,90 g rund 89 %iges *12* und 2,2,5-Trimethyl-8-isobutyl-7-oxa-bicyclo [3.3.0]-octan *12a* im Verhältnis ~ 8 : 1. Eine Probe von *12* wurde mittels präparativer GC nachgereinigt.

IR : 1680, 1062, 1057, 981, 967, 889 $cm^{-1}$

NMR : ~ 0,95 (d, J 6Hz, $2CH_3$-C(9)) ; 1,00 (2s, zusammenfallend, 6H) ; 1,86 (s, 1H-C(6)) ; 4,20 + 4,30 (je m, 1H-C(4), 1H-C(7) ; 4,77 + 4,97 (je s, je 1H-C(11)) δ ppm

MS : 208 ($M^+$, 11), 193 (45), 165 (40), 151 (54), 122 (49), 107 (66), 95 (100), 81 (62), 69 (57), 55 (37), 41 (71) und weitere typische Fragmente bei m/e 123 (43), 109 (54), 93 (42), 79 (36)

## Beispiel 10

9-Aethylmegastigma-5(11), 8-dien-4,7-oxyd *13* und 2,2,5-Trimethyl-8-[2'-methyl-but-1'-enyl]-7-oxa-bicyclo-[3.3.0]-octan *13a*

Analog der Herstellung des 9-Methyl-α-jonols (Beispiel 8) wurden aus 50,0 g (0,26 Mol) α-Jonon und 0,31 Mol Aethylmagnesiumbromid in Aether 59,0 g Rohprodukt erhalten, welches > 90 % (GC) 9-Aethyl-α-jonol enthielt und direkt in dieser Form weiterverarbeitet wurde. Die analog dem Verfahren des Beispiels 4 durchgeführte Epoxydation von 55,7 g (~ 0,25 Mol) rohem 9-Aethyl-α-jonol ergab 57,8 g 90 %iges 9-Aethyl-α-jonolepoxyd, von denen 56,8 g mit 24,5 g (0,12 Mol) Aluminiumisopropylat in 100 ml Isopropanol gelöst wurden. Die Anwendung der im Beispiel 1 beschriebenen Methodik ergab 54,0 g eines Rohproduktes, aus dem durch Destillation über eine 20 cm-Widmer-Kolonne 12,2 g (~ 23 %) olfaktisch gutes Produkt vom Sdp. 73-76°/0,05 mmHg erhalten wurde, das > 90 % *13* und des entsprechenden 7-Oxa-bicyclo [3.3.0]-octan-Derivates, des 2,2,5-Trimethyl-8-[2-methyl-but-1-enyl]-7-oxa-bicyclo [3.3.0]-octans *13a* im Verhältnis von 10 : 1 enthielt.

Spektrale Daten von *13*

IR : 1675, 1052, 1028, 982, 962, 888 $cm^{-1}$

NMR : 0,94 + 1,01 (je s, je 3H) ; 1,0 (t, $CH_3$-C(10)) ; 1,72 (s, $CH_3$-C(9)) ; 1,86 (s, 1H-C(6) ; 4,36 (d, J ~ 4Hz, 1H-C(4)) ; 4,82 (d, J ~ 8Hz, 1H-C(7)) ; 4,76 + 4,94 (je s, je 1H-C(11)) ; 5,13 (d mit Feinaufspaltung, J ~ 8Hz, 1H-C(8)) ; δ ppm

MS : 220, ($M^+$, 8), 137 (11), 122 (77), 107 (100), 99 (35), 93 (36), 79 (18), 69 (7), 55 (10), 41 (17)

Beispiel 11

10-Methylmegastigma-5(11), 8-dien-4,7-oxyd *14* und 2,2,5-Trimethyl-8-[but-1'-enyl]-7-oxa-bicyclo [3.3.0]-octan *14a*

12,0 g (0,054 Mol) n-Methyl-α-jononepoxyd löste man zusammen mit 5,5 g (0,027 Mol) Aluminiumisopropylat in 35 ml Isopropanol und arbeitete analog Beispiel 1. Die Destillation des Rohproduktes (11,5 g) über eine 10 cm-Widmer-Kolonne ergab 2,8 g (= 25 %) olfaktisch gutes Produkt vom Sdp. 60-61°/0,04 mmHg, welches 90 % *14* und 2,2,5-Trimethyl-8-[but-1-enyl]-7-oxa-bicyclo [3.3.0]-octan *14a* im Verhältnis von 10 : 1 enthielt. Eine Probe wurde von *14* mittels präparativer GC nachgereinigt.

Spektrale Daten von *14*

IR : 1685, 1225, 1155, 1053, 988, 970, 960, 890 $cm^{-1}$

NMR : 0,96 + 1,02 (je s, je 3H) ; 0,98 (t, J ~ 7Hz, $CH_3$-C(10)) ; 1,92 (s, 1H-C(6)) ; 4,36 (d, J ~ 4Hz, 1H-C(4)) ; 4,51 (d, J ~ 7Hz, 1H-C(7)) ; 4,76 + 4,96 (je s, je 1H-C(11)) ; 5,2-5,8 (m, 1H-C(8) und 1H-C(9), Signalanalyse vergl. NMR von *1*)

MS : 206 ($M^+$, 1), 122 (56), 107 (100), 93 (40), 91 (26), 81 (22), 79 (41), 77 (20), 67 (20), 55 (27), 41 (38)

Beispiel 12

10-Methylmegastigm-5(11)-en-4,7-oxyd *15* und 2,2,5-Trimethyl-8-butyl-7-oxa-bicyclo [3.3.0]-octan *15a*

1,03 g (5 mMol) *14* wurden in 10 ml Aethanol gelöst und in Anwesenheit von 10 mg Pt-IV-oxyd bis zur Aufnahme von 130 ml Wasserstoff hydriert. Die Kugelrohrdestillation des nach der Aufarbeitung erhaltenen Rohproduktes ergab 0,80 g rund 85 %iges Produkt, enthaltend *15* und 2,2,5-Trimethyl-8-butyl-7-oxa-bicyclo [3.3.0]-octan *15a* im Verhältnis 10 : 1. Eine mittels präparativer GC gereinigte Probe von *15* wies folgende spektrale Daten auf :

IR : 1685, 1155, 1058, 967, 980 $cm^{-1}$

NMR : 0,92 (2s, zusammenfallend, 6H) ; 1,83 (s, 1H-C(6)) ; 4,02 + 4,29 (je m, 1H-C(4) und 1H-C(7)) ; 4,72 + 4,92 (je s, je 1H-C(11) δ ppm

MS : 208 ($M^+$, 18), 193 (82), 165 (51), 151 (84), 122 (60), 109 (53), 107 (80), 95 (100), 81 (58), 69 (29), 41 (62) und weitere typische Fragmente bei m/e 164 (38), 123 (51), 93 (38), 79 (32), 67 (27)

Beispiel 13

10-Allylmegastigma-5(11), 8-dien-4,7-oxyd *16* und 2,2,5-Trimethyl-8-[hexa-1',5'-dienyl]-7-oxa-bicyclo-[3.3.0]-octan *16a*

30,6 g (0,12 Mol) aus handelsüblichem n-Allyl-α-jonon hergestelltes n-Allyl-α-jononepoxyd löste man zusammen mit 12,6 g (0,06 Mol) Aluminiumisopropylat in 100 ml Isopropanol und arbeitete analog Beispiel 1. Die Destillation des Rohproduktes (28,2 g) über eine 10 cm-Widmer-Kolonne ergab 6,3 g (≘ 27 %) olfaktisch gutes Produkt vom Sdp. 78-80°/0,04 mmHg, welches > 90 % *16* und 2,2,5-Trimethyl-8-[hexa-1',5'-dienyl]-7-oxa-bicyclo-[3.3.0]-octan *16a* im Verhältnis 10 : 1 enthielt.

Eine mittels präparativer GC gereinigte Probe von *16* wies folgende spektrale Daten auf.

IR : 1685, 1645, 1225, 1151, 1052, 990, 980, 960, 910, 890 $cm^{-1}$

NMR : 0,90 + 0,97 (je s, je 3H) ; 1,87 (s, 1H-C(6)) ; 2,08 (m, 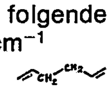 ) ; 4,33 (d, J ~ 4Hz, 1H-C(4)) ; 4,50 (d, j ~ 7Hz, 1H-C(7)) ; 4,72 + 4,92 (je s, je 1H-C(11)) ; 4,8-5,8 (m, 5H) δ ppm

MS : 232 ($M^+$, 1), 122 (56), 107 (100), 93 (54), 91 (38), 81 (19), 79 (42), 77 (25), 67 (15), 55 (16), 41 (36)

Beispiel 14

11-Methylmegastigma-5(11), 8-dien-4,7-oxyd *17* und entsprechendes 7-Oxa-bicyclooctan *17a*

Die säurekatalysierte Umsetzung von 3-Aethyl-7-methyl-oct-1-in-6-en-3-ol mit Isopropenylmethyläther und anschliessende basen-katalysierter Isomerisierung des erhaltenen β-Ketoallenes (G. Saucy, R. Marbet, Helv. *50,* 1158, 1967) lieferte in guter Ausbeute das 6-Aethyl-10-methyl-undeca-3,5,9-trien-2-on, welches auf bekannte Art (H. Rouvé, M. Stoll, Helv. *30,* 2216, (1947)) mit 85 %iger Phosphorsäure cyclisiert wurde. Das erhaltene Produkt enthielt 90 % 11-Methyl-α-jonon und 11-Methyl-γ-jonon (2 Isomere) im Verhältnis von 7 : 3. Analog der Epoxydation von α-Iron (Beispiel 4) wurden aus 13,4 g (0,065 Mol) 11-Methyl-α-jonon 13,9 g rohes 11-Methyl-α-jononepoxyd erhalten, von denen 13,4 g (~ 0,06 Mol) zusammen mit 6,1 g (0,03 Mol) Aluminiumisopropylat in 30 ml Isopropanol gelöst wurden. Die Anwendung der unter Beispiel 1 beschriebenen Methodik ergab 13,7 g eines Rohproduktes, aus dem durch Destillation über eine 5 cm-Widmer-Kolonne 2,10 g (17 %) olfaktisch gutes Produkt von Sdp. 73-75°/0,05 mmHg erhalten wurde, das > 85 % *17* und das entsprechende 7-Oxa-bicyclooctan *17a* enthielt. Eine mittels präparativer GC gereinigte Probe zeigte folgende spektrale Daten.

IR : 1680, 1152, 1050, 877, 962 $cm^{-1}$

NMR : 0,92 + 1,00 (je s, je 3H) ; 1,65 (2 d, überlagert, $CH_3$-C(9) und $CH_3$-C(11)) ; 4,28 (d, J ~ 4Hz, 1H-C(4)) ; 4,47 (d, J ~ 7Hz, 1H-C(7)) ; 5,1-5,9 (m, 1H-C(8), 1H-C(9) und 1H-C(11) δ ppm

MS : 206 ($M^+$, 8), 136 (90), 121 (100), 109 (41), 107 (91), 93 (82), 91 (47), 79 (48), 67 (41), 55 (38), 41 (60)

## Beispiel 15

4,11-Dimethylmegastigma-5(11), 8-dien-4,7-oxyd *18* und entsprechendes 7-Oxa-bicyclooctan *18a*

Die Cyclisierung des 6-Aethyl-7,10-dimethyl-undeca-3,5,9-trien-2-ons (Herstellung analog Beispiel 14) mit 85 %iger Phosphorsäure führte zu einem Rohprodukt, das 90 % 3,11-Dimethyl-α-jonon und 3,11-Dimethyl-γ-jonon (2 Isomere) im ungefähren Verhältnis von 7 : 3 enthielt. Analog der Epoxydation von α-Iron (Beispiel 4) wurden aus 50,0 g (0,23 Mol) 3,11-Dimethyl-α-jonon 54 g rohes 3,11-Dimethyl-α-jononepoxyd erhalten, von denen 49 g (~ 0,21 mol) zusammen mit 21,4 g (0,105 Mol) Aluminiumisopropylat in 80 ml Isopropanol gelöst wurden. Die Anwendung der Methodik des Beispiels 1 ergab 46 g eines Rohproduktes, aus dem durch Destillation über eine 10 cm-Widmer-Kolonne 4,4 g (~ 10 %) olfaktisch gutes Produkt vom Sdp. 72-74°/0,04 mmHg erhalten wurde, das 70 % *18* neben dem entsprechenden 7-Oxa-bicyclooctan *18a* enthielt. Zur Charakterisierung benützte man eine auf säulenchromatographischem Wege gereinigte Probe.

IR : 1680, 1232, 1162, 1155, 1128, 1092, 1026, 980, 965, 952, 935, 840, 820 $cm^{-1}$

NMR : 0,92 + 0,4 (je s, je 3H) ; 1,30 (s, $CH_3$-C(4)) ; 1,66 (2 d, überlagert, J je ~ 7Hz, $CH_3$-C(9) und $CH_3$-C(11)) ; 2,32 (s, 1H-C(6)) ; 4,42 (d, Z ~ 7Hz, 1H-C(7)) ; 5,1-5,8 (m, 1H-C(8), 1H-C(9) und 1H-C(11)) δ ppm

MS : 220 ($M^+$, 29), 177 (97), 149 (57), 135 (87), 121 (89), 109 (100), 107 (78), 91 (48), 81 (32), 55 (30), 43 (81) und weitere typische Fragmente bei m/e 123 (50), 95 (30), 93 (42), 79 (28), 67 (26), 41 (42)

## Beispiel 16

12/13-Methylmegastigma-5(11), 8-dien-4,7-oxyd *19* und entsprechendes 7-Oxa-bicyclooctan *19a*

Die Cyclisierung des 6,10-Dmethyl-dodeca-3,5,9-trien-2-ons (Herstellung analog Beispiel 14) mit 85 %iger Phosphorsäure führte zu einem Rohprodukt, das > 90 % 12/13-Methyl-α-jonon und 12/13-Methyl-β-jonon im ungefähren Verhältnis von 9 : 1 enthielt. Analog der Epoxydation von α-Iron (Beispiel 4) wurden aus 52,7 g (0,256 Mol) 12/13-Methyl-α-jonon 49,0 g (≙ 85 %) 12/13-Methyl-α-jononepoxyd vom Sdp. 109-111°/0,3 mmHg erhalten, von denen 19,2 g (0,086 Mol) zusammen mit 8,8 g (0,043 Mol) Aluminiumisopropylat in 50 ml Isopropanol gelöst wurden. Die Anwendung der im Beispiel 1 beschriebenen Methodik ergab 17,8 g eines Rohproduktes, aus dem durch Destillation über eine 10 cm-Widmer-Kolonne 4,0 g (≙ 17,7 %) olfaktisch gutes Produkt vom Sdp. 70-73°/0,3 mmHg erhalten wurde, welches > 85 % *19* neben dem entsprechenden 7-Oxa-bicyclo-octan *19a* enthielt. Eine mittels präparativer GC gereinigte Probe zeigte folgende spektrale Daten :

IR : 1685, 1220, 1155, 1056, 990, 963, 944, 890 $cm^{-1}$

NMR : ~ 0,90 (t, $CH_3$-C (12/13)) ; 0,92 (s, 3H) ; 1,68 (d, J ~ 6Hz, $CH_3$-C(9)) ; 2,02 (s, 1H-C(6)) ; 4,32 (d, J ~ 4Hz, 1H-C(4)) ; 4,50 (d, J ~ 7Hz, 1H-C(7)) ; 4,80 + 4,97 (je s, je 1H-C(11)) ; 5,2-5,8 (m, 1H-C(8) und 1H-C(9)) δ ppm

MS : 206 ($M^+$, 5), 177 (32), 136 (59), 121 (50), 107 (100), 93 (50), 91 (40), 79 (49), 67 (16), 55 (25), 41 (41)

## Beispiel 17

9-Vinylmegastigma-5(11), 8-dien-4,7-oxyd *20* und entsprechendes 7-Oxa-bicyclooctan *20c*

Zu einer auf 0° gekühlten Grignard-Lösung, welche aus 9,72 g (0,4 Mol) Magnesiumspänen in 75 ml Tetrahydrofuran und 44,9 g (0,42 Mol) Vinylbromid in 180 ml Tetrahydrofuran hergestellt wurde, liess man im Verlaufe von 20 Minuten eine Lösung von 50 g (0,26 Mol) α-Jonon in 150 ml Tetrahydrofuran so zutropfen, dass die Reaktionstemperatur zwischen 15 und 20° lag. Anschliessend rührte man noch während 3 Stunden bei Raumtemperatur und arbeitete gemäss Beispiel 8 auf. Es resultierten 54,5 g Rohprodukt, welches nach GC > 92 % 9-Vinyl-α-jonol enthielt, und welches direkt in dieser Form weiterverarbeitet wurde. Analog der Epoxydation von α-Iron (Beispiel 4) wurden aus 53,2 g (0,242 Mol) 9-Vinyl-α-jonol 59,0 g rohes 9-Vinyl-α-jonolepoxyd erhalten, von denen 58,0 g zusammen mit 26,5 g (0,13 Mol) Aluminiumisopropylat in 175 ml Isopropanol gelöst wurden. Die Anwendung der Methodik des Beispiels 1 ergab 57 g eines Rohproduktes, aus dem durch Destillation über eine 20 cm-Widmer-Kolonne 15,0 g (≙ 26 %) olfaktisch gutes Produkt vom Sdp. 73-74°/0,3 mmHg erhalten wurde, das > 85 % *20a* und *20b* im ungefähren Verhältnis von 3 : 2 enthielt. Daneben sind geringe Mengen des entsprechenden 7-Oxa-bicyclo-octans *20c* vorhanden.

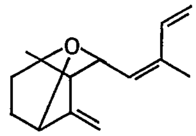

20 a

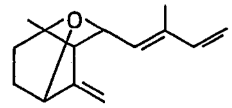

20 b

Spektrale Daten von *20a* und *20b*

*20a.* IR : 1680, 1600, 1052, 990, 980, 960, 905, 890 $cm^{-1}$

NMR : 0,96 + 1,04 (je s, je 3H) ; 1,82 (s, $CH_3$-C(9)) ; 1,92 (s, 1H-C(6)) ; 4,38 (d, J ~ 4Hz, 1H-C(4)) ; 5,04 (d, J ~ 7Hz, 1H-C(7)) ; 4,80 + 4,98 (je s, je 1H-C(11)) ; 5,2 (m, 1H-C(8)) ; 5,1-5,4 (m, 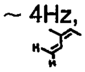 ) ; 6,72-7,02 (m, 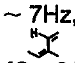 ) δ ppm

MS : 218 ($M^+$, 14), 150 (9), 122 (65), 107 (100), 97 (35), 93 (39), 79 (19), 67 (5), 55 (8), 41 (13)

*20b.* IR : 1680, 1605, 1056, 992, 988, 963, 905, 898 $cm^{-1}$

NMR : 0,98 + 1,04 (je s, je 3H) ; 1,84 (s, $CH_3$-C(9)) ; 1,94 (s, 1H-C(6)) ; 4,40 (d, J ~ 4Hz, 1H-C(4)) ; 4,96 (d, J ~ 7Hz, 1H-C(7)) ; 4,80 + 4,98 (je s, je 1H-C(11)) ; 5,1 (m, 1H-C(8)) ; 5,0-5,5 (m, 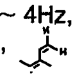 ) ; 6,2-6,5 (m, 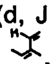 ) δ ppm

MS : 218 ($M^+$, 15), 150 (10), 122 (65), 107 (100), 97 (41), 93 (38), 79 (25), 68 (17), 55 (10), 41 (18)

## Beispiel 18

Parfümerie-Komplexe mit einem Gehalt an Megastigma-5(11), 8-dien-4,7-oxyd *1* (*)

| | Gewichtsteile |
|---|---|
| Geraniol extra | 450 |
| 2-Aethyl-3,6,6-trimethyl-2-cyclohexen-1-ylcarbonsäureäthylester | 450 |
| | 900 |

Gibt man zu diesem Komplex 100 Teile *1*, so wird dieser sehr angenehm abgerundet und wirkt nun an sich schon eher wie eine Base in Richtung Rose. Es entsteht eine sehr würzige Rosennote mit ausgeprägter Diffusion und Fülle.

Aendert man den Komplex dahingehend ab, dass man nur Geraniol mit *1* im Verhältnis 1 : 1 mischt, so entsteht ein verblüffender Effekt in Richtung Rhodinol. Es entsteht eine Rosen-Base mit sehr intensiver Kopfnote, welche sich sehr gut für Rosen-Kompositionen mit sehr moderner Note eignet. Ausserdem resultiert eine Erhöhung der Haftfestigkeit des Geraniols.

---

(*) In diesem — und den folgenden FormulierungsL...pielen — sind die Angaben betr. verwendete Verbindungen I jeweils so zu verstehen, dass als I das nach dem erfindungsgemässen Verfahrens anfallenden Gemisch von I mit wenig II eingesetzt wurde. Anstelle dieses Gemisches kann auch I allein eingesetzt werden.

## Beispiel 19

Parfümerie-Komplex mit einem Gehalt an Megastigma-5(11), 8-dien-4,7-oxyd *1*

| | Gewichtsteile |
|---|---|
| Linalool synthetisch | 200 |
| Geraniol synthetisch | 720 |
| Citral | 50 |
| | 970 |

Gibt man zu diesem frisch-blumigen Komplex 30 Teile Megastigma-5(11), 8-dien-4,7-oxyd, so gewinnt er an angenehmer Frische und wirkt bezüglich Blumennote viel natürlicher. Man hat nun den Eindruck, dass natürliches Rosenöl und Citronenöl bei der Herstellung in kleinen Mengen mitverwendet worden sind.

## Beispiel 20

Parfümerie-Komplex mit einem Gehalt an 2-Methylmegastigma-5(11), 8-dien-4,7-oxyd *5*

| | Gewichtsteile |
|---|---|
| Bergamotteöl | 200 |
| Sandelholzöl | 200 |
| Methyldihydrojasmonat | 200 |
| | 600 |

Dieser Komplex, der in der Luxus-Parfümerie Anwendung finden kann, wirkt nach Zusatz von 400 Teilen *5* sehr viel weicher, harmonischer und natürlicher. Das neue *5* verbindet die Sandelholzöl-Methyldihydrojasmonat-Note geradezu ideal. Es entsteht ein Komplex, der sehr vornehm wirkt. Setzt man die Konzentration von *5* herab und gibt z.B. nur 100 Teile zu, so entsteht ein Komplex ganz anderer Richtung. Die Zitrus-Richtung ändert sich. Während in der ursprünglichen Base das Bergamotteöl dominierte, wirkt der Komplex nun eher in Richtung Zitronenöl.

Beispiel 21

Parfümerie-Komplex Richtung Holz mit einem Gehalt an 8-Methyl-megastigma-5(11), 8-dien-4,7-oxyd *7*

| | Gewichtsteile |
|---|---|
| Sandelholzöl | 340 |
| Patchouliöl | 340 |
| Vetiveröl | 200 |
| Cedrylacetat | 60 |
| | 940 |

Gibt man zu diesem « Holz-Komplex » 60 Teile *7*, so wird er ausgesprochen in Richtung Sandelholz verändert. Er erweckt den Eindruck von frisch geschnittenem, jungem Holz. Der neue Komplex ist kräftiger und diffusiver.

Beispiel 22

Parfümerie-Base mit einem Gehalt an Megastigma-5(11), 8-dien-4,7-oxyd *1*

| | Gewichtsteile |
|---|---|
| Propylenglykol | 190 |
| Phenyläthylalkohol | 400 |
| Geraniol extra | 100 |
| Nerol | 100 |
| Cinnamylpropionat | 70 |
| Zimtalkohol | 60 |
| α-Jonon | 20 |
| 4-Acetyl-6-tert.-butyl-1,1-dimethyl-indan | 10 |
| Rosenoxyd 10 % in Propylenglykol | 10 |
| Geranium Bourbonöl 10 % in Propylenglykol | 10 |
| | 970 |

Gibt man zu dieser Rosen-Base 30 Teile *1*, so wird diese steigender, fruchtiger, die Geranium-Note wird eindrucksvoll unterstrichen und es ist nun eine viel typischere Rose entstanden. Das Gleichgewicht zwischen Geraniumöl und Geraniol ist in der neuen Base so ausgeglichen, dass ein sehr schöner Rhodinol-Effekt entsteht.

Beispiel 23

Parfümerie-Komposition Richtung Tee mit einem Gehalt an Megastigma-5(11), 8-dien-4,7-oxyd *1*

| | Gewichtsteile |
|---|---|
| Bergamotteöl | 240 |
| Hydroxycitronellal | 120 |
| Methyldihydrojasmonat | 120 |
| Patchouliblätteröl | 60 |
| Basilikumöl | 60 |
| Cedrylacetat crist. | 60 |
| Acetanisol | 40 |
| β-Jonon | 40 |
| 12-Oxa-hexadecanolid | 40 |
| Baum-Moos abs. 50 % in Propylenglykol | 20 |
| Zitronenöl | 20 |
| Acetyliertes Zedernholzöl | 20 |
| Beifussöl | 20 |
| Kamillenöl römisch 10 % in Propylenglykol | 20 |
| Indol 10 % in Propylenglykol | 10 |

| | |
|---|---|
| Rhodinol | 10 |
| Thibetolide (omega-Pentadecalacton) | 10 |
| Fichtennadel-balsam absolut | 6 |
| Wurmsamenöl | 4 |
| Methyleugenol | 60 |
| | 980 |

Gibt man zu dieser Parfümerie-Komposition 20 Teile *1*, so kommt ein erwünschter Tee-Effekt zur Geltung. Die Komposition wirkt blumiger, kräftiger, aromatischer. Der Kamillen-Charakter wird ausgesprochen unterstrichen. *1* zeigt in dieser Komposition einen sehr guten, harmonisierenden und verbindenden Effekt.

## Beispiel 24

Parfümerie-Komposition allgemein blumiger Richtung mit einem Gehalt an Megastigma-5(11), 8-dien-4,7-oxyden [*1* bzw. *7*] bzw. den Oxa-bicyclo [3.3.0]-octanen [*2* bzw. *8*]

| | Gewichtsteile |
|---|---|
| Phenyläthyl-phenylacetat | 150 |
| Benzylsalicylat | 130 |
| Methyl-1-methylcyclododecyläther | 120 |
| Glycerylacetal des Phenylacetaldehyds | 100 |
| Sandelholzöl | 100 |
| Hydroxycitronellal | 80 |
| Aethylenbrassylat | 30 |
| Linalool | 30 |
| Eugenol extra | 10 |
| Zyklamenaldehyd | 10 |
| Phenyläthylalkohol | 100 |
| Methyldihydrojasmonat | 100 |
| | 960 |

Gibt man zu dieser blumigen Komposition 40 Teile *1*, so wirkt die entstandene Base frisch-blumig, viel leichter, « sauberer » und ist sehr geeignet für moderne, jugendliche Linien.

Durch Zusatz von 40 Teilen 8-Methylmegastigma-5(11), 8-dien-4,7-oxyd *7* wird die Komposition andererseits viel schwerer, süsser und weist eine ausgeprägte Nelken-Note auf.

Ein Zusatz von 40 Teilen *2* verleiht der Komposition eine angenehme würzige und rosenartige Note, welche frisch und belebend wirkt.

Durch Zusatz von 40 Teilen *8* wird die Komposition allgemein blumiger Richtung überraschenderweise in Richtung Rose verändert.

## Beispiel 25

Parfümerie-Komposition Richtung Chypre mit einem Gehalt an Megastigma-5(11), 8-dien-4,7-oxyden [*1*, *5*, *7* bzw. *14*]

| | Gewichtsteile |
|---|---|
| Methyl-1-methylcyclododecyläther | 200 |
| a-Hexylzimtaldehyd | 200 |
| Bergamotteöl | 200 |
| Methyldihydrojasmonat | 80 |
| Vetiverylacetat | 60 |
| α-Jonon | 20 |
| Corianderöl | 20 |
| Benzylacetat | 20 |
| Rhodinol | 20 |
| Aethylenbrassylat | 20 |
| 8α,12-Oxido-13,14,15,16-tetranorlabdan | 10 |
| Wurmsamenöl | 10 |
| p-Menthan-8-thiol-3-on | 10 |
| 3,5-Dimethylcyclohex-3-en-1-ylcarboxaldehyd 10 % in Propylenglykol | 10 |
| Baumoos absolue 50 % in Propylenglykol | 20 |
| Patchouliöl | 20 |
| | 920 |

Gibt man zu dieser Chyprekomposition 80 Teile Megastigma-5(11), 8-dien-4,7-oxyd *1*, erhält man eine

sehr ansprechende würzig-herbe, und gleichzeitig auch blumige Kopfnote, die sich sehr für die moderne Parfümerie (Herren-Linien) eignet.

Durch Zusatz von 80 Teilen 2-Methyl-megastigma-5(11), 8-dien-4,7-oxyd *5* erhält man eine sehr angenehm warme, weiche Edelholz-Note, welche in Luxus-Parfums ausgesprochen gefragt ist. Die Komposition weist eine sehr elegante Note auf.

Durch Zusatz von 40 Teilen 8-Methyl-megastigma-5(11), 8-dien-4,7-oxyd *7* wird die Komposition wesentlich holziger, aber auch frischer und würziger ; geeignet für Herren-Noten.

Ein Zusatz von 40 Teilen 10-Methyl-megastigma-5(11), 8-dien-4,7-oxyd *14* führt in Richtung des warmen, schönholzigen Chypre, aber mit wesentlich mehr Diffusion als dies in der ursprünglichen Komposition der Fall ist.

### Beispiel 26

Parfümerie-Komposition Richtung Tabak mit einem Gehalt an Megastigma-5(11), 8-dien-4,7-oxyden [*1*, *14* bzw. *7*]

| | Gewichtsteile |
|---|---|
| α-Jonon | 200 |
| o-tertiär-Butylcyclohexylacetat | 200 |
| a-Hexyl-zimtaldehyd | 110 |
| Keton-moschus | 100 |
| Sandelholzöl | 100 |
| Styrallylacetat | 60 |
| Methyldihydrojasmonat | 60 |
| Cumarin | 20 |
| Benzoe resinoid | 20 |
| Isobutylchinolin 10 % in Propylenglykol | 20 |
| Lavendelöl | 20 |
| Vetiveröl Bourbon | 10 |
| Melilotus absolue farblos | 10 |
| Galbanumöl | 10 |
| | 940 |

Durch Zusatz von 6 % *1* erhält die obige Komposition eine sehr originelle Kopfnote, die wesentlich frischer wirkt. Die so entstandene Komposition ist geeignet für moderne Tabak-Noten z.B. in Herren-Linien. Durch Zusatz von 6 % von 10-Methyl-megastigma-5(11), 8-dien-4,7-oxyd *14* erzielt man hingegen den typischen Geruch der klassischen « Tabak-Parfümerie ». Die neue Komposition wirkt viel wärmer, voller und besitzt einen typischeren Tabak-Charakter.

Durch Zusatz von nur 2 % von 8-Methylmegastigma-5(11), 8-dien-4,7-oxyd *7* wird eine eher holzige, sehr frische Komposition erhalten, ausgesprochen geeignet für Herren-Linien.

### Beispiel 27

Fruchtige Parfümerie-Base mit einem Gehalt an 2-Methyl-megastigma-5(11), 8-dien-4,7-oxyd *5*

| | Gewichtsteile |
|---|---|
| Aethylphthalat | 370 |
| Methyldihydrojasmonat | 200 |
| [4-(4-Methyl-3-pentenyl)-3-cyclohexen-1-yl]-methylacetat | 120 |
| Cyklamenaldehyd | 80 |
| 2,6-Dimethyl-6-hepten-1-al, 10 % in Propylenglykol | 60 |
| 2-Methyl-1,3-dioxolan-2-äthylacetat | 60 |
| cis-6-Nonenol, 10 % in Propylenglykol | 40 |
| cis-3-Hexenylacetat, 10 % in Propylenglykol | 20 |
| Zitronenöl | 20 |
| | 970 |

Ein Zusatz von 30 Teilen *5* verleiht der Fruchtbase viel mehr Natürlichkeit. Der Melonen-Charakter wird verstärkt, die süss-fruchtige Note kommt besser zur Geltung.

### Beispiel 28

Grüne Parfümerie-Base mit einem Gehalt an 2-Methyl-megastigma-5(11), 8-dien-4,7-oxyd *5*

| | Gewichtsteile |
|---|---|
| Methyldihydrojasmonat | 300 |
| Bergamotteöl | 300 |

| | |
|---|---|
| a-Hexylzimtaldehyd | 200 |
| Basilikumöl | 40 |
| Linalylanthranilat | 20 |
| 3,5-Dimethylcyclohex-3-en-1-ylcarboxaldehyd, 10 % in Propylenglykol | 20 |
| p-Menthan-8-thiol-3-on | 10 |
| Galbanumöl | 10 |
| Propylenglykol | 20 |
| | 920 |

Die etwas hart-grüne Base gewinnt durch Zusatz von 80 Teilen *5* einen viel natürlicheren Ton. Die Komposition erinnert ausgesprochen an den in einem Blumengeschäft bzw. in einem Treibhaus vorherrschenden Geruch. Der Komplex wirkt weich, rund, nach jungen Blättern, er erinnert an helles « Grün ».

Beispiel 29

Himbeeraroma mit einem Gehalt an Megastigma-5(11), 8-dien-4,7-oxyd *1*

| | Gewichtsteile |
|---|---|
| cis-3-Hexenol | 0,15 |
| Methylanthranilat | 0,15 |
| 4-(p-Hydroxyphenyl)-2-butanon | 0,5 |
| Dimethylsulfid | 0,15 |
| Citral | 0,1 |
| Bernsteinsäurediäthylester | 1,5 |
| γ-Undecalacton | 1,5 |
| Selleriesamenöl | 1,0 |
| Anethol | 2,0 |
| Aethylisovalerat | 2,5 |
| Aethyl-α-methylphenylglycidat | 3,0 |
| Vanillin | 4,0 |
| Aethylacetat | 6,0 |
| Jasmin absolue 10 % in Aethanol | 2,0 |
| β-Jonon | 50,0 |
| Aethanol | 915,45 |
| | 990,0 |

Durch Zusatz von 10 Teilen einer 1 %igen Lösung von *1* in Aethanol wird obiges Himbeeraroma in vorteilhafter Weise verändert. Geruchlich und geschmacklich tritt nun die schwache holzig-frische, ausgeprägt fruchtig-süsse Note, die für reife Himbeeren charakteristisch ist, in Erscheinung.

Beispiel 30

Teearoma mit einem Gehalt an Megastigma-5(11), 8-dien-4,7-oxyd *1*

| | Gewichtsteile |
|---|---|
| Isovaleraldehyd | 0,5 |
| Campher, 10 % in Aethanol | 0,5 |
| Geraniol, 10 % in Aethanol | 1,0 |
| Vanillin | 1,0 |
| Linalylacetat, 1 % in Aethanol | 2,0 |
| Linalool | 2,0 |
| Butylalkohol | 2,0 |
| Citral, 1 % in Aethanol | 3,0 |
| Tannin, 5 % in Wasser | 100,0 |
| Propylenglykol | 838,0 |
| | 950,0 |

Versetzt man diese Teebase mit 50 Teilen einer 1 %igen Lösung von *1* in Aethanol, ist sofort eine vorteilhafte Veränderung feststellbar. Geruchlich und geschmacklich tritt die holzige, herb-frische Note, die für Schwarztee charakteristisch ist, deutlich in Erscheinung.

Beispiel 31

Megastigma-5(11), 8-dien-4,7-oxyd *1* als Tabakzusatz

100 g Maryland-Tabak wurden gleichmässig mit 4 ml einer 1 %igen Lösung von *1* in Aethanol besprüht und anschliessend während 24 Stunden bei Raumtemperatur gelagert. Die aus dem so

behandelten Tabak hergestellten Zigaretten zeigten beim Verrauch einen angenehmen Kühleffekt. Der Rauch wies einen originellen fruchtigblumigen Aspekt auf ; er wirkte bedeutend milder und angenehmer als der Rauch der unbehandelten Blindproben.

Beispiel 32

2-Methylmegastigma-5(11), 8-dien-4,7-oxyd *5* als Tabakzusatz

100 g Maryland-Tabak wurden wie in Beispiel 31 beschrieben mit 4 ml einer 1 %igen Lösung von *5* in Aethanol behandelt. Die aus dem so behandelten Tabak hergestellten Zigaretten zeigten beim Verrauchen ein sehr angenehmes, bedeutend weicheres Aroma als die entsprechenden Blindproben.

**Ansprüche**

1. Verbindungen der allgemeinen Formel

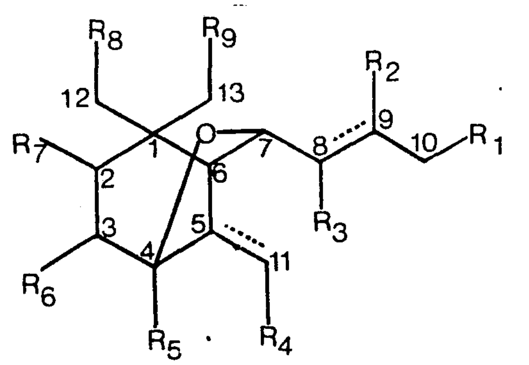

I

worin $R_1$ und $R_2$ Wasserstoff, Methyl, Propyl, Vinyl, Propenyl oder Allyl und $R_3$, $R_4$, $R_5$, $R_6$, $R_7$, $R_8$, $R_9$ Wasserstoff, Methyl oder Aethyl darstellen, und die gestrichelten Linien eine fakultative Bindung, bzw. im Falle von $R_1$ und/oder $R_2$ = Propenyl, Vinyl oder Allyl eine obligatorische Bindung darstellen, und die 5,11-Bindung nur gesättigt ist, wenn der Rest des Moleküls gesättigt ist.

2. Megastigma-5(11), 8-dien-4,7-oxydgemäss Anspruch 1.

3. 8-Methyl-megastigma-5(11), 8-dien-4,7-oxydgemäss Anspruch 1.

4. Eine Verbindung gemäss Anspruch 1, ausgewählt unter 10-Methyl-megastigma-5(11), 8-dien-4,7-oxyd, 10-Allyl-megastigma-5(11), 8-dien-4,7-oxyd, 11-Methyl-megastigma-5(11), 8-dien-4,7-oxyd, 4,11-Dimethyl-megastigma-5(11), 8-dien-4,7-oxyd, 12/13-Methyl-megastigma-5(11), 8-dien-4,7-oxyd und 2-Methyl-megastigma-5(11), 8-dien-4,7-oxyd.

5. Verbindungen der allgemeinen Formel

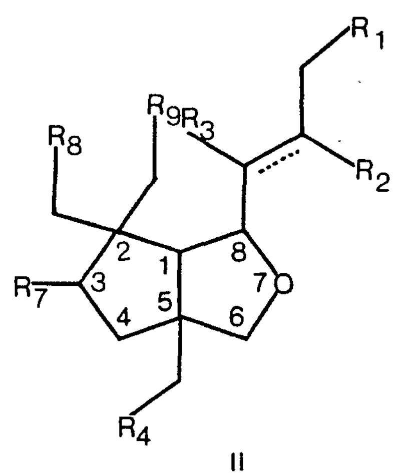

II

worin $R_1$ und $R_2$ Wasserstoff, Methyl, Propyl, Vinyl, Propenyl oder Allyl und $R_3$, $R_4$, $R_5$, $R_6$, $R_7$, $R_8$, $R_9$ Wasserstoff, Methyl oder Aethyl darstellen, und die gestrichelten Linien eine fakultative Bindung, bzw. im Falle von $R_1$ und/oder $R_2$ = Propenyl, Vinyl oder Allyl eine obligatorische Bindung darstellen.

6. 2,2,5-Trimethyl-8-[prop-1'-enyl]-7-oxa-bicyclo-[3.3.0]-octangemäss Anspruch 5.

7. 2,2,5-Trimethyl-8-[1'-methyl-prop-1'-enyl]-7-oxabicyclo [3.3.0]-octangemäss Anspruch 5.

8. Verbindungen der allgemeinen Formel I und/oder II als Riech- und/oder Geschmackstoffe.

9. Riech- und/oder Geschmackstoffkomposition, gekennzeichnet durch einen Gehalt an Verbindungen der allgemeinen Formeln I und/oder II gemäss Ansprüchen 1 und 5.

10. Riech- und/oder Geschmackstoffkomposition gemäss Anspruch 9, gekennzeichnet durch einen Gehalt an Megastigma-5(11), 8-dien-4,7-oxyd und/oder 2,2,5-Trimethyl-8-[prop-1'-enyl]-7-oxabicyclo [3.3.0]-octan.

11. Riech- und/oder Geschmackstoffkomposition gemäss Anspruch 9, gekennzeichnet durch einen Gehalt an 8-Methyl-megastigma-5(11), 8-dien-4,7-oxyd und/oder 2,2,5-Trimethyl-8-[1'-methyl-prop-1'-enyl]-7-oxa-bicyclo [3.3.0]-octan.

12. Verfahren zur Herstellung von Verbindungen der Formeln I und II gemäss Ansprüchen 1 und 5 dadurch gekennzeichnet, dass man eine Verbindung der Formeln

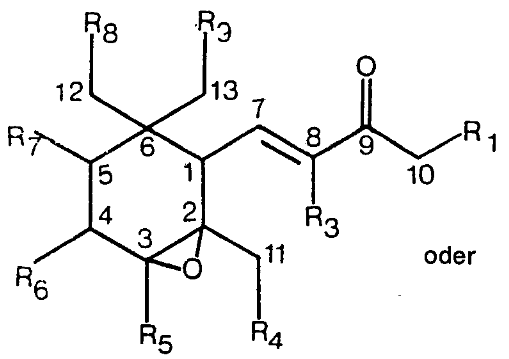

oder

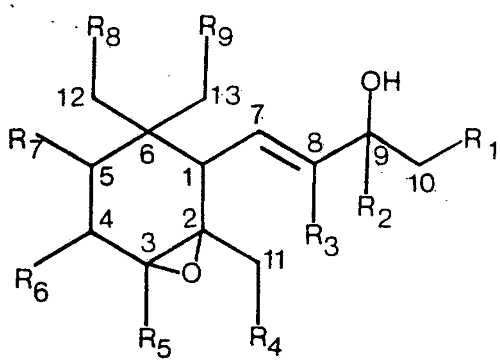

III IV

worin $R_1$ bis $R_9$ die angegebene Bedeutung besitzen, mit einem Aluminium-sec.-alkoholat, im Falle von III unter reduzierenden Bedingungen, behandelt, und, gewünschtenfalls, in beliebiger Reihenfolge, das erhaltene Gemisch der Verbindungen der Formeln I und II in die Komponenten auftrennt und in den Verbindungen I und II allenfalls vorhandene C = C-Bindungen hydriert.

13. Verfahren nach Anspruch 12, dadurch gekennzeichnet, dass man Aluminiumisopropylat verwendet.

14. Verwendung von Verbindungen der allgemeinen Formeln I und/oder II gemäss Ansprüchen 1 und 5 als Riech- und/oder Geschmackstoffe.

## Claims

1. Compounds of the general formula

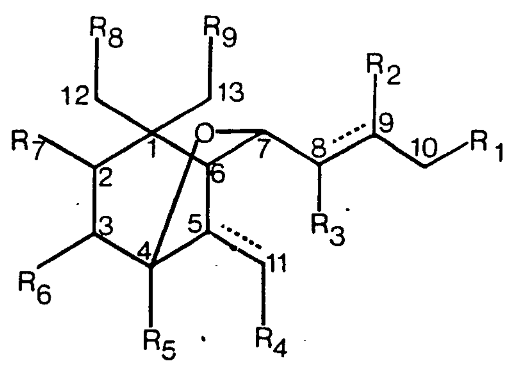

I

wherein $R_1$ and $R_2$ represent a hydrogen atom or a methyl, propyl, vinyl, propenyl or allyl group and $R_3$, $R_4$, $R_5$, $R_6$, $R_7$, $R_8$ and $R_9$ each represent a hydrogen atom or a methyl or ethyl group, and the dotted lines represent an optional bond or, when $R_1$ and/or $R_2$ represents a propenyl, vinyl or allyl group, an obligatory bond, and the 5,11-bond is only saturated when the remainder of the molecule is saturated.

2. Megastigma-5(11), 8-dien-4,7-oxide according to claim 1.

3. 8-Methyl-megastigma-5(11), 8-dien-4,7-oxide according to claim 1.

4. A compound according to claim 1 selected from 10-methyl-megastigma-5(11), 8-dien-4,7-oxide, 10-allyl-megastigma-5(11), 8-dien-4,7-oxide, 11-methyl-megastigma-5(11), 8-dien-4,7-oxide, 4,11-dimethyl-megastigma-5(11), 8-dien-4,7-oxide, 12/13-methylmegastigma-5(11), 8-dien-4,7-oxide and 2-methyl-megastigma-5(11), 8-dien-4,7-oxide.

5. Compounds of the general formula

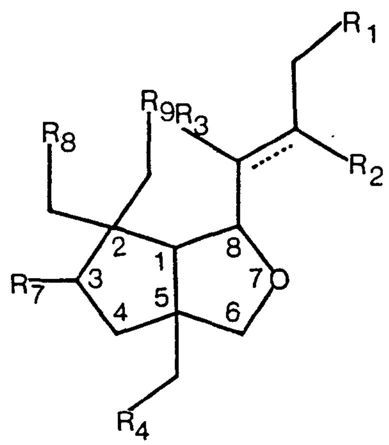

II

wherein $R_1$ and $R_2$ represent a hydrogen atom or a methyl, propyl, vinyl, propenyl or allyl group and $R_3$, $R_4$, $R_5$, $R_6$, $R_7$, $R_8$ and $R_9$ represent a hydrogen atom or a methyl or ethyl group, and the dotted lines represent an optional bond or, when $R_1$ and/or $R_2$ represents a propenyl, vinyl or allyl group, an obligatory bond.

6. 2,2,5-Trimethyl-8-[prop-1′-enyl]-7-oxa-bicyclo [3.3.0]-octane according to claim 5.

7. 2,2,5-Trimethyl-8-[1′-methyl-prop-1′-enyl]-7-oxabicyclo [3.3.0]-octane according to claim 5.

8. Compounds of the general formula I and/or II when used as odorant and/or flavouring substances.

9. An odorant and/or flavouring composition, characterized in that it contains a compound of the general formula I and/or II according to claims 1 and 5.

10. An odorant and/or flavouring composition according to claim 9, characterized in that it contains megastigma-5(11), 8-dien-4,7-oxide and/or 2,2,5-trimethyl-8-[prop-1′-enyl]-7-oxa-bicyclo [3.3.0]-octane.

11. An odorant and/or flavouring composition according to claim 9, characterized in that it contains 8-methyl-megastigma-5(11), 8-dien-4,7-oxide and/or 2,2,5-trimethyl-8-[1′-methyl-prop-1′-enyl]-7-oxa-bicyclo-[3.3.0]-octane.

12. A process for the manufacture of compounds of the general formulae I and II according to claims 1 and 5, characterized by treating a compound of the formulae

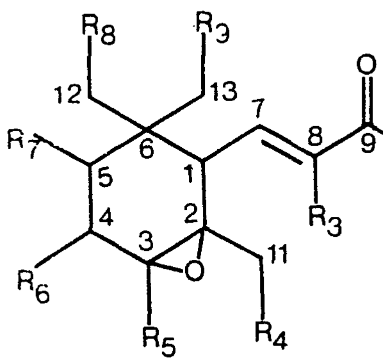

or

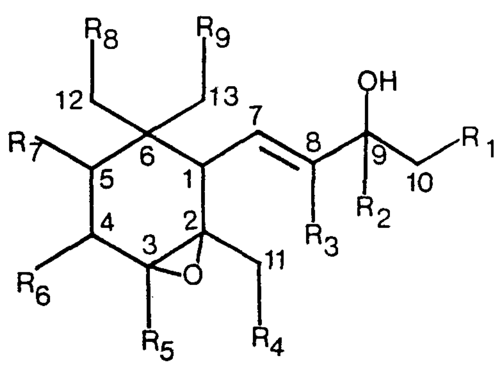

III IV

wherein $R_1$ to $R_9$ have the significance given earlier, with an aluminium sec. alcoholate, the treatment being carried out under reducing conditions when a compound of formula III is used, and, if desired, in optional sequence, separating the resulting mixture of the compounds of formulae I and II into the components and hydrogenating C = C bonds which may be present in the compounds of formulae I and II.

13. A process according to claim 12, wherein aluminium isopropylate is used as the aluminium sec. alcoholate.

14. Use of compounds of the general formulae I and/or II according to claims 1 and 5 as odorant and/or flavouring substances.

**Revendications**

1. Composés de formule générale

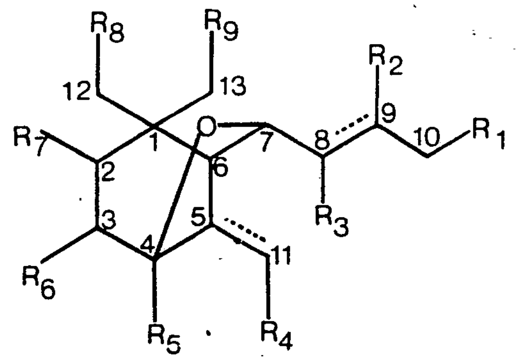

I

dans laquelle $R_1$ et $R_2$ représentent un atome d'hydrogène ou un groupe méthyle, propyle, vinyle, propényle ou allyle et $R_3$, $R_4$, $R_5$, $R_6$, $R_7$, $R_8$ et $R_9$ représentent chacun un atome d'hydrogène ou un groupe méthyle ou éthyle, et les lignes pointillées représentent une liaison facultative ou, lorsque $R_1$ et/ou $R_2$ représente un groupe propényle, vinyle ou allyle, une liaison obligatoire, et la liaison 5,11 est seulement saturée lorsque le reste de la molécule est saturé.

2. Mégastigma-5(11), 8-diène-4,7-oxyde selon la revendication 1.

3. 8-Méthyl-mégastigma-5(11), 8-diène-4,7-oxyde selon la revendication 1.

4. Un composé selon la revendication 1, sélectionné parmi le 10-méthyl-mégastigma-5(11), 8-diène-4,7-oxyde, 10-allyl-métastigma-5(11), 8-diène-4,7-oxyde, 11-méthyl-mégastigma-5(11), 8-diène-4,7-oxyde, 4,11-diméthyl-mégastigma-5(11), 8-diène-4,7-oxyde, 12/13-méthyl-mégastigma-5(11), 8-diène-4,7-oxyde et le 2-méthyl-mégastigma-5(11), 8-diène-4,7-oxyde.

5. Composés de formule générale

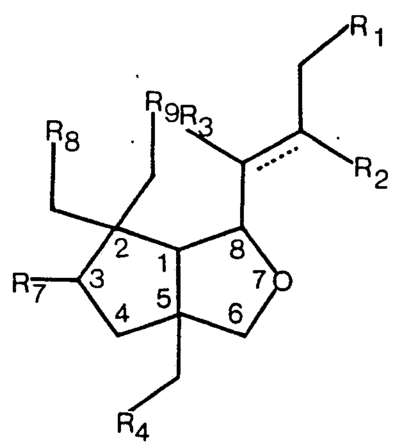

II

dans laquelle $R_1$ et $R_2$ représentent un atome d'hydrogène ou un groupe méthyle, propyle, vinyle, propényle ou allyle et $R_3$, $R_4$, $R_5$, $R_6$, $R_7$, $R_8$ et $R_9$ représentent chacun un atome d'hydrogène ou un groupe méthyle ou éthyle, et les lignes pointillées représentent une liaison facultative ou, lorsque $R_1$ et/ou $R_2$ représentent un groupe propényle, vinyle ou allyle, une liaison obligatoire.

6. 2,2,5-Triméthyl-8-[prop-1'-ényl]-7-oxa-bicyclo-[3.3.0]-octane selon la revendication 5.

7. 2,2,5-Triméthyl-8-[1'-méthyl-prop-1'-ényl]-7-oxabicyclo [3.3.0]-octane selon la revendication 5.

8. Composés de formule générale I et/ou II comme odorants et/ou substances d'assaisonnement.

9. Une composition odorante et/ou d'assaisonnement, caractérisée par une teneur en un composé de formule générale I et/ou II selon les revendications 1 et 5.

10. Une composition odorante et/ou d'assaisonnement selon la revendication 9, caractérisée par une teneur en mégastigma-5(11), 8-diène-4,7-oxyde et/ou 2,2,5-triméthyl-8-[prop-1'-ényl]-7-oxa-bicyclo [3.3.0]-octane.

11. Une composition odorante et/ou d'assaisonnement selon la revendication 9, caractérisée par une teneur en 8-méthyl-mégastigma-5(11), 8-diène-4,7-oxyde et/ou 2,2,5-triméthyl-8-[1'-méthyl-prop-1'-ényl]-7-oxa-bicyclo-[3.3.0]-octane.

12. Un procédé pour la préparation de composés de formules générales I et II selon les revendications 1 et 5, caractérisé en ce qu'on traite un composé de formules

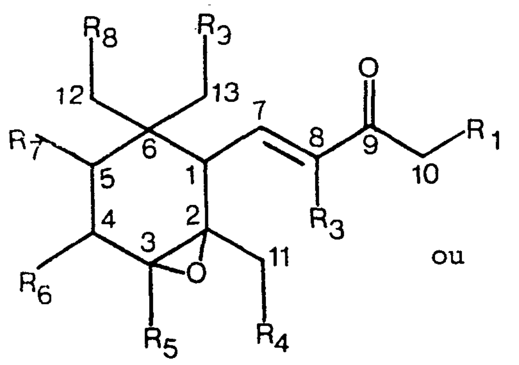

III

ou

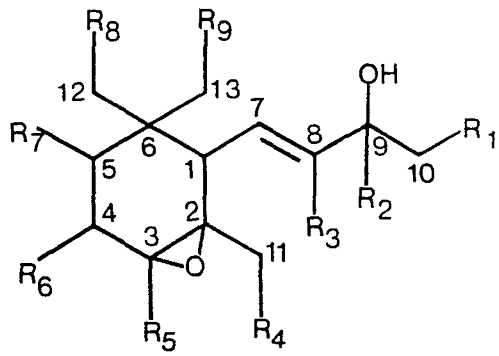

IV

dans lesquelles $R_1$ à $R_9$ ont la signification donnée, avec un sec. alcoolate d'aluminium, dans le cas de III sous conditions réductrices, et, le cas échéant, dans une séquence quelconque, on résoud le mélange obtenu de composés de formules I et II en les constituants individuels et hydrogène les liaisons C = C pouvant être présentes dans les composés I et II.

13. Un procédé selon la revendication 12, caractérisé en ce qu'on utilise l'isopropylate d'aluminium.

14. L'utilisation de composés de formules générales I et/ou II selon les revendications 1 et 5 comme odorants et/ou substances d'assaisonnement.